# EUROPEAN PATENT SPECIFICATION

(11) **EP 3 211 007 B1**
(45) Date of publication and mention of the grant of the patent: **28.07.2021**
(21) Application number: 17157844.6
(22) Date of filing: 24.02.2017
(51) Int. Cl.: C07K 16/24, A61K 39/00

(54) **PHARMACEUTICAL COMPOSITION FOR TREATING CROHN'S DISEASE**
PHARAMZEUTISCHEZUSAMMENSETZUNG ZUR BEHANDLUNG VON MORBUS CROHN
COMPOSITIONS ET MÉTHODES POUR LE TRAITEMENT DE MORBUS CROHN

(30) Priority: 29.02.2016 JP 2016036604
(43) Date of publication of application: 30.08.2017
(73) Proprietor: Eisai R&D Management Co., Ltd., Tokyo 112-8088 (JP)
(72) Inventor: Yasuda, Nobuyuki, Ibaraki, 300-2635 (JP); Oketani, Kiyoshi, Tokyo, 112-8088 (JP); Baba, Hiroko, Tokyo, 112-8088 (JP); Nakano, Tomohisa, Tokyo, 112-8088 (JP); Mori, Masahiko, Tokyo, 112-8088 (JP)
(74) Representative: Hoffmann Eitle

(56) References cited:
- WO-A1-2011/052799
- H TABUCHI ET AL: "FIRST-IN-HUMAN (FIH) STUDY OF E6011, A NOVEL HUMANIZED ANTI-FRACTALKINE (CX3CL1) MONOCLONAL ANTIBODY", ANNUAL MEETING OF THE AMERICAN-SOCIETY-FOR-CLINICAL-PHARMACOLOGY -AND-THERAPEUTICS (ASCPT); SAN DIEGO, CA, USA, vol. 99, 1 February 2016 (2016-02-01), page s33, XP055344618,
- TANAKA YOSHIYA ET AL: "Safety and Efficacy of E6011, an Anti-Fractalkine Monoclonal Antibody, in a First-in-Patient Phase 1/2 Study in Rheumatoid Arthritis", ARTHRITIS & RHEUMATOLOGY (HOBOKEN), JOHN WILEY & SONS, INC, US, vol. 67, no. Suppl.10, 1 October 2015 (2015-10-01), XP009193437, ISSN: 2326-5191

### SEQUENCE LISTING

<110> EISAI R&D MANAGEMENT CO., LTD.
<120> A pharmaceutical composition for treating Crohn's disease
<130> ESAP1507-EP
<160> 26
<170> PatentIn version 3.5
<210> 1
   <211> 119
   <212> PRT
   <213> Artificial Sequence
<220>
   <223> Synthetic polypeptide
<400> 1
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asn Tyr
               20                  25                  30
   Tyr Ile His Trp Val Lys Gln Ala Pro Gly Gln Gly Leu Glu Trp Ile
           35                  40                  45
   Gly Trp Ile Tyr Pro Gly Asp Gly Ser Pro Lys Phe Asn Glu Arg Phe
       50                  55                  60
   Lys Gly Arg Thr Thr Leu Thr Ala Asp Lys Ser Thr Asn Thr Ala Tyr
   65                  70                  75                  80
   Met Leu Leu Ser Ser Leu Arg Ser Glu Asp Thr Ala Val Tyr Phe Cys
                   85                  90                  95
   Ala Thr Gly Pro Thr Asp Gly Asp Tyr Phe Asp Tyr Trp Gly Gln Gly
               100                 105                 110
   Thr Thr Val Thr Val Ser Ser
           115
<210> 2
   <211> 107
   <212> PRT
   <213> Artificial Sequence
<220>
   <223> Synthetic polypeptide
<400> 2
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gly Asn Ile His Asn Phe
               20                  25                  30
   Leu Ala Trp Tyr Gln Gln Lys Pro Gly Lys Ala Pro Lys Leu Leu Ile
           35                  40                  45
   Tyr Asn Glu Lys Thr Leu Ala Asp Gly Val Pro Ser Arg Phe Ser Gly
       50                  55                  60
   Ser Gly Ser Gly Thr Asp Tyr Thr Leu Thr Ile Ser Ser Leu Gln Pro
   65                  70                  75                  80
   Glu Asp Phe Ala Thr Tyr Phe Cys Gln Gln Phe Trp Ser Thr Pro Tyr
                   85                  90                  95
   Thr Phe Gly Gly Gly Thr Lys Val Glu Ile Lys
               100                 105
<210> 3
   <211> 19
   <212> PRT
   <213> Artificial Sequence
<220>
   <223> Synthetic polypeptide
<400> 3
   Met Glu Trp Ser Trp Val Phe Leu Phe Phe Leu Ser Val Thr Thr Gly
   1               5                   10                  15
   Val His Ser
<210> 4
   <211> 326
   <212> PRT
   <213> Artificial Sequence
<220>
   <223> Synthetic polypeptide
<400> 4
   Ala Ser Thr Lys Gly Pro Ser Val Phe Pro Leu Ala Pro Cys Ser Arg
   1               5                   10                  15
   Ser Thr Ser Glu Ser Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Asn Phe Gly Thr Gln Thr
   65                  70                  75                  80
   Tyr Thr Cys Asn Val Asp His Lys Pro Ser Asn Thr Lys Val Asp Lys
                   85                  90                  95
   Thr Val Glu Arg Lys Cys Cys Val Glu Cys Pro Pro Cys Pro Ala Pro
               100                 105                 110
   Pro Ala Ala Ala Pro Ser Val Phe Leu Phe Pro Pro Lys Pro Lys Asp
           115                 120                 125
   Thr Leu Met Ile Ser Arg Thr Pro Glu Val Thr Cys Val Val Val Asp
       130                 135                 140
   Val Ser His Glu Asp Pro Glu Val Gln Phe Asn Trp Tyr Val Asp Gly
   145                 150                 155                 160
   Val Glu Val His Asn Ala Lys Thr Lys Pro Arg Glu Glu Gln Phe Asn
                   165                 170                 175
   Ser Thr Phe Arg Val Val Ser Val Leu Thr Val Val His Gln Asp Trp
               180                 185                 190
   Leu Asn Gly Lys Glu Tyr Lys Cys Lys Val Ser Asn Lys Gly Leu Pro
           195                 200                 205
   Ala Pro Ile Glu Lys Thr Ile Ser Lys Thr Lys Gly Gln Pro Arg Glu
       210                 215                 220
   Pro Gln Val Tyr Thr Leu Pro Pro Ser Arg Glu Glu Met Thr Lys Asn
   225                 230                 235                 240
   Gln Val Ser Leu Thr Cys Leu Val Lys Gly Phe Tyr Pro Ser Asp Ile
                   245                 250                 255
   Ala Val Glu Trp Glu Ser Asn Gly Gln Pro Glu Asn Asn Tyr Lys Thr
               260                 265                 270
   Thr Pro Pro Met Leu Asp Ser Asp Gly Ser Phe Phe Leu Tyr Ser Lys
               275                 280                 285
   Leu Thr Val Asp Lys Ser Arg Trp Gln Gln Gly Asn Val Phe Ser Cys
       290                 295                 300
   Ser Val Met His Glu Ala Leu His Asn His Tyr Thr Gln Lys Ser Leu
   305                 310                 315                 320
   Ser Leu Ser Pro Gly Lys
                   325
<210> 5
   <211> 464
   <212> PRT
   <213> Artificial Sequence
<220>
   <223> Synthetic polypeptide
<400> 5
   Met Glu Trp Ser Trp Val Phe Leu Phe Phe Leu Ser Val Thr Thr Gly
   1               5                   10                  15
   Val His Ser Gln Val Gln Leu Val Gln Ser Gly Ala Glu Val Lys Lys
               20                  25                  30
   Pro Gly Ala Ser Val Lys Val Ser Cys Lys Ala Ser Gly Tyr Thr Phe
           35                  40                  45
   Thr Asn Tyr Tyr Ile His Trp Val Lys Gln Ala Pro Gly Gln Gly Leu
       50                  55                  60
   Glu Trp Ile Gly Trp Ile Tyr Pro Gly Asp Gly Ser Pro Lys Phe Asn
   65                  70                  75                  80
   Glu Arg Phe Lys Gly Arg Thr Thr Leu Thr Ala Asp Lys Ser Thr Asn
                   85                  90                  95
   Thr Ala Tyr Met Leu Leu Ser Ser Leu Arg Ser Glu Asp Thr Ala Val
               100                 105                 110
   Tyr Phe Cys Ala Thr Gly Pro Thr Asp Gly Asp Tyr Phe Asp Tyr Trp
           115                 120                 125
   Gly Gln Gly Thr Thr Val Thr Val Ser Ser Ala Ser Thr Lys Gly Pro
       130                 135                 140
   Ser Val Phe Pro Leu Ala Pro Cys Ser Arg Ser Thr Ser Glu Ser Thr
   145                 150                 155                 160
   Ala Ala Leu Gly Cys Leu Val Lys Asp Tyr Phe Pro Glu Pro Val Thr
                   165                 170                 175
   Val Ser Trp Asn Ser Gly Ala Leu Thr Ser Gly Val His Thr Phe Pro
               180                 185                 190
   Ala Val Leu Gln Ser Ser Gly Leu Tyr Ser Leu Ser Ser Val Val Thr
           195                 200                 205
   Val Pro Ser Ser Asn Phe Gly Thr Gln Thr Tyr Thr Cys Asn Val Asp
       210                 215                 220
   His Lys Pro Ser Asn Thr Lys Val Asp Lys Thr Val Glu Arg Lys Cys
   225                 230                 235                 240
   Cys Val Glu Cys Pro Pro Cys Pro Ala Pro Pro Ala Ala Ala Pro Ser
                   245                 250                 255
   Val Phe Leu Phe Pro Pro Lys Pro Lys Asp Thr Leu Met Ile Ser Arg
               260                 265                 270
   Thr Pro Glu Val Thr Cys Val Val Val Asp Val Ser His Glu Asp Pro
           275                 280                 285
   Glu Val Gln Phe Asn Trp Tyr Val Asp Gly Val Glu Val His Asn Ala
       290                 295                 300
   Lys Thr Lys Pro Arg Glu Glu Gln Phe Asn Ser Thr Phe Arg Val Val
   305                 310                 315                 320
   Ser Val Leu Thr Val Val His Gln Asp Trp Leu Asn Gly Lys Glu Tyr
                   325                 330                 335
   Lys Cys Lys Val Ser Asn Lys Gly Leu Pro Ala Pro Ile Glu Lys Thr
               340                 345                 350
   Ile Ser Lys Thr Lys Gly Gln Pro Arg Glu Pro Gln Val Tyr Thr Leu
           355                 360                 365
   Pro Pro Ser Arg Glu Glu Met Thr Lys Asn Gln Val Ser Leu Thr Cys
       370                 375                 380
   Leu Val Lys Gly Phe Tyr Pro Ser Asp Ile Ala Val Glu Trp Glu Ser
   385                 390                 395                 400
   Asn Gly Gln Pro Glu Asn Asn Tyr Lys Thr Thr Pro Pro Met Leu Asp
   405 410 415
   Ser Asp Gly Ser Phe Phe Leu Tyr Ser Lys Leu Thr Val Asp Lys Ser
   420 425 430
   Arg Trp Gln Gln Gly Asn Val Phe Ser Cys Ser Val Met His Glu Ala
   435 440 445
   Leu His Asn His Tyr Thr Gln Lys Ser Leu Ser Leu Ser Pro Gly Lys
   450 455 460
<210> 6
   <211> 20
   <212> PRT
   <213> Artificial Sequence
<220>
   <223> Synthetic polypeptide
<400> 6
   Met Ser Val Pro Thr Gln Val Leu Gly Leu Leu Leu Leu Trp Leu Thr
   1 5 10 15
   Asp Ala Arg Cys
   20
<210> 7
   <211> 107
   <212> PRT
   <213> Artificial Sequence
<220>
   <223> Synthetic polypeptide
<400> 7
<210> 8
   <211> 234
   <212> PRT
   <213> Artificial Sequence
<220>
   <223> Synthetic polypeptide
<400> 8
   Met Ser Val Pro Thr Gln Val Leu Gly Leu Leu Leu Leu Trp Leu Thr
   1               5                   10                  15
   Asp Ala Arg Cys Asp Ile Gln Met Thr Gln Ser Pro Ser Ser Leu Ser
               20                  25                  30
   Ala Ser Val Gly Asp Arg Val Thr Ile Thr Cys Arg Ala Ser Gly Asn
           35                  40                  45
   Ile His Asn Phe Leu Ala Trp Tyr Gln Gln Lys Pro Gly Lys Ala Pro
       50                  55                  60
   Lys Leu Leu Ile Tyr Asn Glu Lys Thr Leu Ala Asp Gly Val Pro Ser
   65                  70                  75                  80
   Arg Phe Ser Gly Ser Gly Ser Gly Thr Asp Tyr Thr Leu Thr Ile Ser
                   85                  90                  95
   Ser Leu Gln Pro Glu Asp Phe Ala Thr Tyr Phe Cys Gln Gln Phe Trp
               100                 105                 110
   Ser Thr Pro Tyr Thr Phe Gly Gly Gly Thr Lys Val Glu Ile Lys Arg
           115                 120                 125
   Thr Val Ala Ala Pro Ser Val Phe Ile Phe Pro Pro Ser Asp Glu Gln
       130                 135                 140
   Leu Lys Ser Gly Thr Ala Ser Val Val Cys Leu Leu Asn Asn Phe Tyr
   145                 150                 155                 160
   Pro Arg Glu Ala Lys Val Gln Trp Lys Val Asp Asn Ala Leu Gln Ser
                   165                 170                 175
   Gly Asn Ser Gln Glu Ser Val Thr Glu Gln Asp Ser Lys Asp Ser Thr
               180                 185                 190
   Tyr Ser Leu Ser Ser Thr Leu Thr Leu Ser Lys Ala Asp Tyr Glu Lys
           195                 200                 205
   His Lys Val Tyr Ala Cys Glu Val Thr His Gln Gly Leu Ser Ser Pro
       210                 215                 220
   Val Thr Lys Ser Phe Asn Arg Gly Glu Cys
   225                 230
<210> 9
   <211> 1417
   <212> DNA
   <213> Artificial Sequence
<220>
   <223> Synthetic oligonucleotide
<400> 9
   aagcttgccg ccaccatgga atggtcctgg gtgttcctgt tcttcctgtc cgtgaccacc 60
   ggcgtgcact cccaggtgca gctggtgcag tctggcgccg aagtgaagaa acctggcgcc 120
   tccgtgaagg tgtcctgcaa ggcctccggc tacaccttca ccaactacta catccactgg 180
   gtgaaacagg ccccaggaca gggcctggaa tggatcggct ggatctaccc cggcgacggc 240
   tcccccaagt tcaacgagcg gttcaagggc cggaccaccc tgaccgccga caagtccacc 300
   aacaccgcct acatgctgct gtcctccctg cggagcgagg ataccgccgt gtacttctgc 360
   gccaccggcc ctaccgacgg cgactacttc gactactggg gccagggcac caccgtgacc 420
   gtgtcctctg cctccaccaa gggcccctcc gtgttccctc tggccccttg ctcccggtcc 480
   acctctgagt ctaccgccgc tctgggctgc ctggtcaaag actacttccc cgagcctgtg 540
   acagtgtcct ggaactctgg cgccctgacc tctggagtgc ataccttccc tgccgtgctg 600
   cagtcatccg gcctgtactc cctgtcctcc gtggtgacag tgccctcctc caacttcggc 660
   acccagacct acacctgtaa cgtggaccac aagccctcca acaccaaggt ggacaagacc 720
   gtggaacgga agtgctgcgt ggaatgcccc ccctgtcctg cccctcctgc cgccgctcct 780
   tccgtgtttc tgttcccccc aaagcccaag gacaccctga tgatctcccg gacccccgaa 840
   gtgacctgcg tggtggtgga cgtgtcccac gaggaccccg aggtgcagtt caattggtac 900
   gtggacggcg tggaagtgca caacgccaag accaagccca gagaggaaca gttcaactcc 960
   accttccggg tggtgtccgt gctgaccgtg gtgcaccagg actggctgaa cggcaaagag 1020
   tacaagtgca aggtctccaa caagggcctg cctgccccca tcgaaaagac catcagcaag 1080
   accaagggcc agccccgcga gccccaggtg tacacactgc cccccagccg ggaagagatg 1140
   accaagaacc aggtgtccct gacctgtctg gtgaaaggct tctacccctc cgatatcgcc 1200
   gtggaatggg agtccaacgg acagcccgag aacaactaca agaccacccc ccccatgctg 1260
   gactccgacg gctcattctt cctgtactcc aagctgacag tggacaagtc ccggtggcag 1320
   cagggcaacg tgttctcctg cagcgtgatg cacgaggccc tgcacaacca ctacacccag 1380
   aagtccctga gcctgagccc cggcaagtga tgaattc 1417
<210> 10
   <211> 727
   <212> DNA
   <213> Artificial Sequence
<220>
   <223> Synthetic oligonucleotide
<400> 10
   aagcttgccg ccaccatgtc cgtgcccacc caggtgctgg gcctgctgct gctgtggctg      60
   accgacgcca gatgcgacat ccagatgacc cagtccccct ccagcctgtc cgcctctgtg     120
   ggcgacagag tgaccatcac ctgtcgggcc tccggcaaca tccacaactt tctggcctgg     180
   tatcagcaga agcccggcaa ggcccccaag ctgctgatct acaacgaaaa gaccctggcc     240
   gacggcgtgc cctccagatt ctccggctct ggctccggca ccgactacac cctgaccatc     300
   tccagcctgc agcccgagga cttcgccacc tacttttgcc agcagttctg gtccaccccc     360
   tacaccttcg gcggaggcac caaggtggaa atcaagcgga ccgtggccgc tccctccgtg     420
   ttcatcttcc caccctccga cgagcagctg aagtccggca ccgcctccgt ggtgtgcctg     480
   ctgaacaact tctacccccg cgaggccaag gtgcagtgga aggtggacaa cgccctgcag     540
   tccggcaact cccaggaatc cgtcaccgag caggactcca aggacagcac ctactccctg     600
   tcctccaccc tgaccctgtc caaggccgac tacgagaagc acaaggtgta cgcctgcgaa     660
   gtgacccacc agggcctgtc cagccccgtg accaagtcct tcaaccgggg cgagtgctga     720
   tgaattc                                                               727
<210> 11
   <211> 445
   <212> PRT
   <213> Artificial Sequence
<220>
   <223> Synthetic polypeptide
<400> 11
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asn Tyr
               20                  25                  30
   Tyr Ile His Trp Val Lys Gln Ala Pro Gly Gln Gly Leu Glu Trp Ile
           35                  40                  45
   Gly Trp Ile Tyr Pro Gly Asp Gly Ser Pro Lys Phe Asn Glu Arg Phe
       50                  55                  60
   Lys Gly Arg Thr Thr Leu Thr Ala Asp Lys Ser Thr Asn Thr Ala Tyr
   65                  70                  75                  80
   Met Leu Leu Ser Ser Leu Arg Ser Glu Asp Thr Ala Val Tyr Phe Cys
                   85                  90                  95
   Ala Thr Gly Pro Thr Asp Gly Asp Tyr Phe Asp Tyr Trp Gly Gln Gly
               100                 105                 110
   Thr Thr Val Thr Val Ser Ser Ala Ser Thr Lys Gly Pro Ser Val Phe
           115                 120                 125
   Pro Leu Ala Pro Cys Ser Arg Ser Thr Ser Glu Ser Thr Ala Ala Leu
       130                 135                 140
   Gly Cys Leu Val Lys Asp Tyr Phe Pro Glu Pro Val Thr Val Ser Trp
   145                 150                 155                 160
   Asn Ser Gly Ala Leu Thr Ser Gly Val His Thr Phe Pro Ala Val Leu
                   165                 170                 175
   Gln Ser Ser Gly Leu Tyr Ser Leu Ser Ser Val Val Thr Val Pro Ser
               180                 185                 190
   Ser Asn Phe Gly Thr Gln Thr Tyr Thr Cys Asn Val Asp His Lys Pro
           195                 200                 205
   Ser Asn Thr Lys Val Asp Lys Thr Val Glu Arg Lys Cys Cys Val Glu
       210                 215                 220
   Cys Pro Pro Cys Pro Ala Pro Pro Ala Ala Ala Pro Ser Val Phe Leu
   225                 230                 235                 240
   Phe Pro Pro Lys Pro Lys Asp Thr Leu Met Ile Ser Arg Thr Pro Glu
                   245                 250                 255
   Val Thr Cys Val Val Val Asp Val Ser His Glu Asp Pro Glu Val Gln
               260                 265                 270
   Phe Asn Trp Tyr Val Asp Gly Val Glu Val His Asn Ala Lys Thr Lys
           275                 280                 285
   Pro Arg Glu Glu Gln Phe Asn Ser Thr Phe Arg Val Val Ser Val Leu
   290 295 300
   Thr Val Val His Gln Asp Trp Leu Asn Gly Lys Glu Tyr Lys Cys Lys
   305 310 315 320
   Val Ser Asn Lys Gly Leu Pro Ala Pro Ile Glu Lys Thr Ile Ser Lys
   325 330 335
   Thr Lys Gly Gln Pro Arg Glu Pro Gln Val Tyr Thr Leu Pro Pro Ser
   340 345 350
   Arg Glu Glu Met Thr Lys Asn Gln Val Ser Leu Thr Cys Leu Val Lys
   355 360 365
   Gly Phe Tyr Pro Ser Asp Ile Ala Val Glu Trp Glu Ser Asn Gly Gln
   370 375 380
   Pro Glu Asn Asn Tyr Lys Thr Thr Pro Pro Met Leu Asp Ser Asp Gly
   385 390 395 400
   Ser Phe Phe Leu Tyr Ser Lys Leu Thr Val Asp Lys Ser Arg Trp Gln
   405 410 415
   Gln Gly Asn Val Phe Ser Cys Ser Val Met His Glu Ala Leu His Asn
   420 425 430
   His Tyr Thr Gln Lys Ser Leu Ser Leu Ser Pro Gly Lys
   435 440 445
<210> 12
   <211> 214
   <212> PRT
   <213> Artificial Sequence
<220>
   <223> Synthetic polypeptide
<400> 12
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gly Asn Ile His Asn Phe
   20 25 30
   Leu Ala Trp Tyr Gln Gln Lys Pro Gly Lys Ala Pro Lys Leu Leu Ile
   35 40 45
   Tyr Asn Glu Lys Thr Leu Ala Asp Gly Val Pro Ser Arg Phe Ser Gly
   50 55 60
   Ser Gly Ser Gly Thr Asp Tyr Thr Leu Thr Ile Ser Ser Leu Gln Pro
   65 70 75 80
   Glu Asp Phe Ala Thr Tyr Phe Cys Gln Gln Phe Trp Ser Thr Pro Tyr
   85 90 95
   Thr Phe Gly Gly Gly Thr Lys Val Glu Ile Lys Arg Thr Val Ala Ala
   100 105 110
   Pro Ser Val Phe Ile Phe Pro Pro Ser Asp Glu Gln Leu Lys Ser Gly
   115 120 125
   Thr Ala Ser Val Val Cys Leu Leu Asn Asn Phe Tyr Pro Arg Glu Ala
   130 135 140
   Lys Val Gln Trp Lys Val Asp Asn Ala Leu Gln Ser Gly Asn Ser Gln
   145 150 155 160
   Glu Ser Val Thr Glu Gln Asp Ser Lys Asp Ser Thr Tyr Ser Leu Ser
   165 170 175
   Ser Thr Leu Thr Leu Ser Lys Ala Asp Tyr Glu Lys His Lys Val Tyr
   180 185 190
   Ala Cys Glu Val Thr His Gln Gly Leu Ser Ser Pro Val Thr Lys Ser
   195 200 205
   Phe Asn Arg Gly Glu Cys
   210
<210> 13
   <211> 119
   <212> PRT
   <213> Artificial Sequence
<220>
   <223> Synthetic polypeptide
<400> 13
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Asn Tyr
   20 25 30
   Tyr Ile His Trp Val Lys Gln Ala Pro Gly Gln Gly Leu Glu Trp Ile
           35                  40                  45
   Gly Trp Ile Tyr Pro Gly Asp Gly Ser Pro Lys Phe Asn Glu Arg Phe
       50                  55                  60
   Lys Gly Arg Thr Thr Leu Thr Ala Asp Lys Ser Thr Asn Thr Ala Tyr
   65                  70                  75                  80
   Met Leu Leu Ser Ser Leu Arg Ser Glu Asp Thr Ala Val Tyr Phe Cys
                   85                  90                  95
   Ala Thr Gly Pro Thr Asp Gly Asp Tyr Phe Asp Tyr Trp Gly Gln Gly
               100                 105                 110
   Thr Thr Val Thr Val Ser Ser
           115
<210> 14
   <211> 107
   <212> PRT
   <213> Artificial Sequence
<220>
   <223> Synthetic polypeptide
<400> 14
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gly Asn Ile His Asn Phe
               20                  25                  30
   Leu Ala Trp Tyr Gln Gln Lys Pro Gly Lys Ala Pro Lys Leu Leu Ile
           35                  40                  45
   Tyr Asn Glu Lys Thr Leu Ala Asp Gly Val Pro Ser Arg Phe Ser Gly
       50                  55                  60
   Ser Gly Ser Gly Thr Asp Tyr Thr Leu Thr Ile Ser Ser Leu Gln Pro
   65                  70                  75                  80
   Glu Asp Phe Ala Thr Tyr Phe Cys Gln Gln Phe Trp Ser Thr Pro Tyr
                   85                  90                  95
   Thr Phe Gly Gly Gly Thr Lys Val Glu Ile Lys
               100                 105
<210> 15
   <211> 5
   <212> PRT
   <213> Artificial Sequence
<220>
   <223> Synthetic polypeptide
<400> 15
   Asn Tyr Tyr Ile His
   1               5
<210> 16
   <211> 17
   <212> PRT
   <213> Artificial Sequence
<220>
   <223> Synthetic polypeptide
<400> 16
   Trp Ile Tyr Pro Gly Asp Gly Ser Pro Lys Phe Asn Glu Arg Phe Lys
   1               5                   10                  15
   Gly
<210> 17
   <211> 10
   <212> PRT
   <213> Artificial Sequence
<220>
   <223> Synthetic polypeptide
<400> 17
   Gly Pro Thr Asp Gly Asp Tyr Phe Asp Tyr
   1               5                   10
<210> 18
   <211> 11
   <212> PRT
   <213> Artificial Sequence
<220>
   <223> Synthetic polypeptide
<400> 18
   Arg Ala Ser Gly Asn Ile His Asn Phe Leu Ala
   1               5                   10
<210> 19
   <211> 7
   <212> PRT
   <213> Artificial Sequence
<220>
   <223> Synthetic polypeptide
<400> 19
   Asn Glu Lys Thr Leu Ala Asp
   1               5
<210> 20
   <211> 9
   <212> PRT
   <213> Artificial Sequence
<220>
   <223> Synthetic polypeptide
<400> 20
   Gln Gln Phe Trp Ser Thr Pro Tyr Thr
   1               5
<210> 21
   <211> 119
   <212> PRT
   <213> Artificial Sequence
<220>
   <223> Synthetic polypeptide
<400> 21
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asn Tyr
               20                  25                  30
   Tyr Ile His Trp Val Lys Gln Ala Pro Gly Gln Gly Leu Glu Trp Ile
           35                  40                  45
   Gly Trp Ile Tyr Pro Gly Asp Gly Ser Pro Lys Phe Asn Glu Arg Phe
       50                  55                  60
   Lys Gly Arg Thr Thr Leu Thr Ala Asp Lys Ser Thr Asn Thr Ala Tyr
   65                  70                  75                  80
   Met Leu Leu Ser Ser Leu Arg Ser Asp Asp Thr Ala Val Tyr Phe Cys
                   85                  90                  95
   Ala Thr Gly Pro Thr Asp Gly Asp Tyr Phe Asp Tyr Trp Gly Gln Gly
               100                 105                 110
   Thr Thr Val Thr Val Ser Ser
   115
<210> 22
   <211> 119
   <212> PRT
   <213> Artificial Sequence
<220>
   <223> Synthetic polypeptide
<400> 22
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Asn Tyr
   20 25 30
   Tyr Ile His Trp Val Arg Gln Ala Pro Gly Gln Gly Leu Glu Trp Ile
   35 40 45
   Gly Trp Ile Tyr Pro Gly Asp Gly Ser Pro Lys Phe Asn Glu Arg Phe
   50 55 60
   Lys Gly Arg Thr Thr Leu Thr Arg Asp Lys Ser Thr Asn Thr Ala Tyr
   65 70 75 80
   Met Glu Leu Ser Ser Leu Arg Ser Asp Asp Thr Ala Val Tyr Phe Cys
   85 90 95
   Ala Thr Gly Pro Thr Asp Gly Asp Tyr Phe Asp Tyr Trp Gly Gln Gly
   100 105 110
   Thr Thr Val Thr Val Ser Ser
   115
<210> 23
   <211> 119
   <212> PRT
   <213> Artificial Sequence
<220>
   <223> Synthetic polypeptide
<400> 23
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Asn Tyr
   20 25 30
   Tyr Ile His Trp Val Arg Gln Ala Pro Gly Gln Gly Leu Glu Trp Ile
           35                  40                  45
   Gly Trp Ile Tyr Pro Gly Asp Gly Ser Pro Lys Phe Asn Glu Arg Phe
       50                  55                  60
   Lys Gly Arg Thr Thr Met Thr Ala Asp Thr Ser Thr Ser Thr Ala Tyr
   65                  70                  75                  80
   Met Glu Leu Ser Ser Leu Arg Ser Glu Asp Thr Ala Val Tyr Phe Cys
                   85                  90                  95
   Ala Arg Gly Pro Thr Asp Gly Asp Tyr Phe Asp Tyr Trp Gly Gln Gly
               100                 105                 110
   Thr Thr Val Thr Val Ser Ser
           115
<210> 24
   <211> 119
   <212> PRT
   <213> Artificial Sequence
<220>
   <223> Synthetic polypeptide
<400> 24
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asn Tyr
               20                  25                  30
   Tyr Ile His Trp Val Arg Gln Ala Pro Gly Gln Gly Leu Glu Trp Ile
           35                  40                  45
   Gly Trp Ile Tyr Pro Gly Asp Gly Ser Pro Lys Phe Asn Glu Arg Phe
       50                  55                  60
   Lys Gly Arg Thr Thr Leu Thr Ala Asp Lys Ser Thr Ser Thr Ala Tyr
   65                  70                  75                  80
   Met Glu Leu Ser Ser Leu Arg Ser Glu Asp Thr Ala Val Tyr Phe Cys
                   85                  90                  95
   Ala Arg Gly Pro Thr Asp Gly Asp Tyr Phe Asp Tyr Trp Gly Gln Gly
               100                 105                 110
   Thr Thr Val Thr Val Ser Ser
               115
<210> 25
   <211> 107
   <212> PRT
   <213> Artificial Sequence
<220>
   <223> Synthetic polypeptide
<400> 25
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gly Asn Ile His Asn Phe
               20                  25                  30
   Leu Ala Trp Tyr Gln Gln Lys Pro Gly Lys Ala Pro Lys Phe Leu Val
           35                  40                  45
   Tyr Asn Glu Lys Thr Leu Ala Asp Gly Val Pro Ser Arg Phe Ser Gly
       50                  55                  60
   Ser Gly Ser Gly Thr Gln Tyr Thr Leu Thr Ile Ser Ser Leu Gln Pro
   65                  70                  75                  80
   Glu Asp Phe Ala Thr Tyr Phe Cys Gln Gln Phe Trp Ser Thr Pro Tyr
                   85                  90                  95
   Thr Phe Gly Gly Gly Thr Lys Val Glu Ile Lys
               100                 105
<210> 26
   <211> 107
   <212> PRT
   <213> Artificial Sequence
<220>
   <223> Synthetic polypeptide
<400> 26
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gly Asn Ile His Asn Phe
               20                  25                  30
   Leu Ala Trp Tyr Gln Gln Lys Pro Gly Lys Ala Pro Lys Leu Leu Ile
           35                  40                  45
   Tyr Asn Glu Lys Thr Leu Ala Asp Gly Val Pro Ser Arg Phe Ser Gly
       50                  55                  60
   Ser Gly Ser Gly Thr Gln Tyr Thr Leu Thr Ile Ser Ser Leu Gln Pro
   65                  70                  75                  80
   Glu Asp Phe Ala Thr Tyr Phe Cys Gln Gln Phe Trp Ser Thr Pro Tyr
                   85                  90                  95
   Thr Phe Gly Gly Gly Thr Lys Val Glu Ile Lys
               100                 105

## Description

### Field of the Invention

The present application claims priority from Japanese Patent Application No. 2016-036604, filed on February 29, 2016.

The present invention relates to a pharmaceutical composition for use in treating Crohn's disease, comprising an anti-fractalkine antibody.

### Description of the Related Art

Fractalkine (which is also referred to as "FKN") is a membrane-bound chemokine that is expressed on the surface of a vascular endothelial cell by inflammatory stimulation of LPS, TNF-α, IL-1 or the like. Cells that express an FKN receptor, CX3CR1, bind to the membrane-bound FKN without mediation of selectin or integrin, and cause strong cell adhesion. In addition, secretory FKN shedding from the membrane-bound FKN exhibits a cell migration activity on NK cells, T cells and monocytes having CX3CR1.

The expression of FKN is induced by proinflammatory cytokine on the surface of a vascular endothelial cell. It has been reported that an increase in the expression of FKN and accumulation of CX3CR1⁺ cytotoxic effector lymphocytes and macrophages are observed in patients having Crohn's disease (which is also referred to as "CD").

To date, FKN has been considered to be a promising therapeutic target to ulcerative colitis (which is also referred to as "UC") and inflammatory bowel disease (which is also referred to as "IBD") such as CD (Ann. NY Acad. Sci. 2009; 1173: 350-356). Moreover, it has been suggested that an anti-fractalkine antibody that inhibits the interaction of FKN with CX3CR1 be able to treat IBD including Crohn's disease (WO2006/046739). Thus, it has been suggested that an antibody capable of binding to FKN and inhibiting the action of the FKN be effective for the treatment of Crohn's disease.

To date, the present applicant has reported a plurality of mouse anti-human fractalkine (hFKN) monoclonal antibodies (clones 1F3-1, 3A5-2, 1F3, 1G1, 2B2, 3D5, 3H7, 6D1, 7F6, and 5H7-6). In particular, since the clone 3A5-2 has high neutralizing activity, binding affinity and interspecies cross-reactivity to hFKN, it has been humanized and named as "H3-2L4" in WO2011/052799.

It is an object of the present disclosure to provide a pharmaceutical composition comprising an anti-fractalkine antibody (which is also referred to as an "anti-FKN antibody" in the present description) that provides therapeutically effective improvement to Crohn's disease, after it has been administered to a human subject.

It is another object of the present invention to provide a pharmaceutical composition comprising an anti-FKN-Antibody, which is used to provide therapeutically effective improvements to Crohn's disease.

### SUMMARY OF THE INVENTION

The present invention refers to a pharmaceutical composition for use in treating Crohn's disease, which comprises an anti-fractalkine antibody and a pharmaceutically acceptable excipient as described hereinunder in more detail.

### BRIEF DESCRIPTION OF THE DRAWINGS

FIG. 1 shows the mean serum concentration of H3-2L4 over time, after single-dose intravenous administration of the antibody H3-2L4 (0.04 mg/kg, 0.2 mg/kg, 1 mg/kg, 3 mg/kg, 6 mg/kg and 10 mg/kg administration groups);
FIG. 2 shows the mean serum concentration of H3-2L4 over time, after multiple-dose intravenous administration of the antibody H3-2L4 (2 mg/kg, 5 mg/kg and 10 mg/kg administration groups);
FIG. 3 shows the mean serum concentration of total FKN over time, after multiple-dose intravenous administration of the antibody H3-2L4 (2 mg/kg, 5 mg/kg and 10 mg/kg administration groups); and
FIG. 4 shows the CRP of seven subjects in Cohort 3 (10 mg/kg group) over time, until 12 weeks after administration.

### DETAILED DESCRIPTION OF THE PREFERRED EMBODIMENTS

### 1. The present invention refers to:

[1] A pharmaceutical composition for use in treating Crohn's disease, which comprises an anti-fractalkine antibody and a pharmaceutically acceptable excipient, wherein the anti-fractalkine antibody is an antibody comprising: a heavy chain variable region comprising the amino acid sequence shown in SEQ ID NO: 13 (QVQLVQSGAEVKKPGASVKVSCKASGYTFTNYYIHWVKQAPGQGLEWIGWIYPGDG SPKF NERFKGRTTLTADKSTNTAYMLLSSLRSEDTAVYFCATGPTDGDYFDYWGQGTTVTV SS); a light chain variable region comprising the amino acid sequence shown in SEQ ID NO: 14 (DIQMTQSPSSLSASVGDRVTITCRASGNIHNFLAWYQQKPGKAPKLLIYNEKTLAD GVPSRFS GSGSGTDYTLTISSLQPEDFATYFCQQFWSTPYTFGGGTKVEIK); and a constant region of human IgG2 isotype, wherein the Fc region of the constant region of human IgG2 isotype comprises mutations V234A and G237A, and the anti-fractalkine antibody is intravenously administered to a human at a dose of at least 10 mg/kg of human body weight and the mean trough concentration of the anti-fractalkine antibody is 80 mg/mL or more.
[2] In a second embodiment the invention refers to a pharmaceutical composition for use according to embodiment [1], wherein the anti-fractalkine antibody is intravenously administered to a human at a dose of 10 to 15 mg/kg of human body weight.
[3] In a further embodiment the invention refers to a pharmaceutical composition for use according to embodiment [1], wherein the anti-fractalkine antibody is intravenously administered to a human at a dose of 10 mg/kg of human body weight.
[4] In still a further embodiment the invention refers to a pharmaceutical composition for use according to embodiment [1], wherein the anti-fractalkine antibody is intravenously administered to a human at a dose of 15 mg/kg of human body weight.
[5] And in still a further aspect the invention refers to the pharmaceutical composition for use according to any one of embodiments [1] to [4], which is administered by multiple-dose intravenous administration at dosing intervals from once every week to once every two weeks.

### 2. Outline and Definition of the Invention

The present invention relates to a pharmaceutical composition for use in treating Crohn's disease, thereby providing a therapeutically effective improvement to Crohn's disease.

A"therapeutically effective improvement to Crohn's disease" can mean that therapeutically effective improvement is shown in one or more evaluation criteria of Crohn's disease, which have been established in the present technical field. Examples of such evaluation criteria that can be used herein include, but are not limited to, Crohn's disease activity index (also referred to as "CDAI"), The International Organization for the study of Inflammatory Bowel Disease score (also referred to as "IOIBD score"), and Dutch activity index (also referred to as "Dutch-AI"), in which the symptoms of inflammatory bowel disease, such as diarrhea, intestinal pain, common state, parenteral complication, perianal abscess, fistula, fever and weight reduction are shown as activity indexes. In addition, another example of such evaluation criteria is detection of the serum level of C-reactive protein (also referred to as "CRP") in patients.

With regard to severity classification according to CDAI, a value of less than 150 indicates remission, a value from 150 to 219 indicates a mild case of disease activity, a value from 220 to 450 indicates a medium case of disease activity, and a value of more than 450 indicates a severe case of disease activity.

In line with the above classification system, when the CDAI score before treatment is 220 or more, and the CDAI score after treatment is reduced by 70 or more, and more preferably by 100 or more, using the CDAI score before treatment as a baseline, or when the CDAI score after treatment is preferably less than 150 (remission), it means that "therapeutically effective improvement is provided to Crohn's disease."

In one embodiment of the present invention, the "therapeutically effective serum concentration of an anti-FKN antibody" is the mean trough concentration of an anti-FKN antibody that is 80 µg/mL or more.

The pharmaceutical composition comprising an anti-FKN antibody, which is used to provide the serum concentration of the anti-FKN antibody that is therapeutically effective for a human subject, after multiple-dose administration of the pharmaceutical composition to the human subject. The form of the pharmaceutical composition of the present invention is not particularly limited, and it can be typically the form of an injection formulation prepared for intravenous administration. In one embodiment, the pharmaceutical composition for use according to the present invention is used, such that the mean trough concentration of the anti-FKN antibody is 80 µg/mL or more.

In the present invention, the pharmacokinetic properties of a concentration-time curve, such as the maximum observed serum concentration (Cₘₐₓ), the time to reach Cₘₐₓ (Tₘₐₓ), and a**rea** under the serum concentration time curve (AUC), are examined by statistical methods sufficiently established in the field of pharmacokinetics. When the ratio between the population mean of the above described evaluation parameters in a study preparation and the population mean of the above described evaluation parameters in a standard preparation is 0.80 to 1.25, the two preparations are generally considered to be biologically equivalent to each other.

In the present invention, the mean values of the pharmacokinetic parameters, such as Cₘₐₓ or AUC, can be calculated by any one method of the geometric mean, the arithmetical mean, and the median. In the present description, the mean Cₘₐₓ, the mean AUC, the mean trough concentration, and the mean tFNK concentration are indicated with the arithmetical mean value, unless otherwise specified. Even if the mean value as a target is calculated by a method that is different from the method described in the present description, if the mean value calculated by the method described in the present description is within the numerical range described in the claims, the calculated mean value is intended to belong to the claims according to the present invention.

In the present invention, with regard to the dose of the anti-FKN antibody, "a dose" or "at a dose" means the absolute amount of the anti-FKN antibody for a single administration. Accordingly, when the expression "at a dose of 10 mg/kg of human body weight" is used, for example, it means that the anti-FKN antibody is administered in an amount calculated by multiplying 10 mg by human body weight (kg), by administration of one set of, or simultaneously used two or more sets of, the pharmaceutical composition of the present invention.

In the present invention, with regard to the dosing intervals of the anti-FKN antibody, for example, the expression "dosing intervals from once every week to once every two weeks" means a dosing interval from an n^{th} number of administration (wherein n is an integer) to an n+1^{th} number of administration is from one week to two weeks. The dosing interval from the n^{th} number of administration to the n+1^{th} number of administration may be different from a dosing interval from the n+1^{th} number of administration to an n+2^{th} number of administration. For instance, a case where the dosing interval from the first administration to the second administration is 1 week and the dosing interval from the second administration to the third administration is 2 weeks is naturally included in the "dosing intervals from once every week to once every two weeks."

In the present description, the term "human" or "human subject" is used to mean a healthy adult male, and typically, any given human who exhibits Crohn's disease, or the clinical signs and symptoms of any given disease or disorder, which may develop Crohn's disease. In the present invention, the "human" or the "human subject" is preferably a Crohn's disease patient who has not been obtained sufficient therapeutic effects from at least one time of the standard of care (corticosteroid, an immunomodulator and/or an anti-TNF antibody), or who has not had continuous therapeutic effects therefrom, or who has been intolerant thereto.

### 2. Anti-FKN Antibody

The anti-FKN antibody used in accordance to the invention is described in more detail in the example section.

In the present invention, when an anti-FKN antibody is referred to, the anti-FKN antibody means the humanized anti-human fractalkine antibody H3-2L4, or an antibody functionally equivalent thereto. In the present invention, the "functionally equivalent antibody" means an antibody equivalent to the antibody H3-2L4, in terms of at least any one, or preferably two or more of binding affinity to human FKN, neutralizing activity, cross-reactivity, and blood pharmacokinetics.

In the present invention, when an anti-FKN antibody is referred to, the anti-FKN antibody may include an antigen-binding fragment thereof. Such an antigen-binding fragment is not particularly limited, as long as it is a functional and structural fragment of the anti-FKN antibody, which retains bindability to FKN and is not significantly different from a complete antibody thereof in terms of blood pharmacokinetics. Examples of such an antigen-binding fragment of an antibody include, but are not limited to, Fab, Fab', F(ab')₂, Fv, single-stranded Fv (ScFv), their variants, and other modification structures, such as a fusion protein comprising an antibody portion and an immunoglobulin molecule containing an antigen recognition site.

In one embodiment, the anti-FKN antibody of the present invention may be any given antibody comprising the following CDR sequences:
(a) CDR-H1 comprising the amino acid sequence shown in SEQ ID NO: 15 (NYYIH);
(b) CDR-H2 comprising the amino acid sequence shown in SEQ ID NO: 16 (WIYPGDGSPKFNERFKG);
(c) CDR-H3 comprising the amino acid sequence shown in SEQ ID NO: 17 (GPTDGDYFDY);
(d) CDR-L1 comprising the amino acid sequence shown in SEQ ID NO: 18 (RASGNIHNFLA);
(e) CDR-L2 comprising the amino acid sequence shown in SEQ ID NO: 19 (NEKTLAD); and
(f) CDR-L3 comprising the amino acid sequence shown in SEQ ID NO: 20 (QQFWSTPYT).

In another embodiment, the anti-FKN antibody may be an antibody comprising a heavy chain and a light chain, wherein the heavy chain variable region of the antibody comprises the amino acid sequence shown in SEQ ID NO: 21 (QVQLVQSGAEVKKPGASVKVSCKASGYTFTNYYIHWVKQAPGQGLEWIGWIYPGDG SPKFNERFKGRTTLTADKSTNTAYMLLSSLRSDDTAVYFCATGPTDGDYFDYWGQGT TVTVSS), SEQ ID NO: 13 (QVQLVQSGAEVKKPGASVKVSCKASGYTFTNYYIHWVKQAPGQGLEWIGWIYPGDG SPKFNERFKGRTTLTADKSTNTAYMLLSSLRSEDTAVYFCATGPTDGDYFDYWGQGT TVTVSS), SEQ ID NO: 22 (QVQLVQSGAEVKKPGASVKVSCKASGYTFTNYYIHWVRQAPGQGLEWIGWIYPGDG SPKFNERFKGRTTLTRDKSTNTAYMELSSLRSDDTAVYFCATGPTDGDYFDYWGQGT TVTVSS), SEQ ID NO: 23 (QVQLVQSGAEVKKPGASVKVSCKASGYTFTNYYIHWVRQAPGQGLEWIGWIYPGDG SPKFNERFKGRTTMTADTSTSTAYMELSSLRSEDTAVYFCARGPTDGDYFDYWGQGT TVTVSS), or SEQ ID NO: 24 (QVQLVQSGAEVKKPGASVKVSCKASGYTFTNYYIHWVRQAPGQGLEWIGWIYPGDG SPKFNERFKGRTTLTADKSTSTAYMELSSLRSEDTAVYFCARGPTDGDYFDYWGQGT TVTVSS), and the light chain variable region of the antibody comprises the amino acid sequence shown in SEQ ID NO: 25 (DIQMTQSPSSLSASVGDRVTITCRASGNIHNFLAWYQQKPGKAPKFLVYNEKTLAD GVPSRFSGSGSGTQYTLTISSLQPEDFATYFCQQFWSTPYTFGGGTKVEIK), SEQ ID NO: 14 (DIQMTQSPSSLSASVGDRVTITCRASGNIHNFLAWYQQKPGKAPKLLIYNEKTLAD GVPSRFSGSGSGTDYTLTISSLQPEDFATYFCQQFWSTPYTFGGGTKVEIK), or SEQ ID NO: 26

In a preferred embodiment, the anti-FKN antibody may be an antibody comprising a heavy chain and a light chain, wherein the heavy chain variable region of the antibody comprises the amino acid sequence shown in SEQ ID NO: 13 (QVQLVQSGAEVKKPGASVKVSCKASGYTFTNYYIHWVKQAPGQGLEWIGWIYPGDG SPKFNERFKGRTTLTADKSTNTAYMLLSSLRSEDTAVYFCATGPTDGDYFDYWGQGT TVTVSS), and the light chain variable region of the antibody comprises the amino acid sequence shown in SEQ ID NO: 14

The anti-FKN antibody is an antibody comprising a constant region of human IgG2 isotype.

The anti-FKN antibody is an antibody wherein the Fc region of the constant region of human IgG2 isotype comprises mutations V234A and G237A.

In a particularly preferred embodiment of the present invention, the anti-FKN antibody is the antibody H3-2L4, which consists of a heavy chain consisting of the amino acid sequence shown in SEQ ID NO: 11 (QVQLVQSGAEVKKPGASVKVSCKASGYTFTNYYIHWVKQAPGQGLEWIGWIYPGDG SPKFNERFKGRTTLTADKSTNTAYMLLSSLRSEDTAVYFCATGPTDGDYFDYWGQGT TVTVSSASTKGPSVFPLAPCSRSTSESTAALGCLVKDYFPEPVTVSWNSGALTSGVH TFPAVLQSSGLYSLSSVVTVPSSNFGTQTYTCNVDHKPSNTKVDKTVERKCCVECPP CPAPPAAAPSVFLFPPKPKDTLMISRTPEVTCVVVDVSHEDPEVQFNWYVDGVEVHN AKTKPREEQFNSTFRVVSVLTVVHQDWLNGKEYKCKVSNKGLPAPIEKTISKTKGQP REPQVYTLPPSREEMTKNQVSLTCLVKGFYPSDIAVEWESNGQPENNYKTTPPMLDS DGSFFLYSKLTVDKSRWQQGNVFSCSVMHEALHNHYTQKSLSLSPGK) and a light chain consisting of the amino acid sequence shown in SEQ ID NO: 12

An antibody, which is obtained by appropriately modifying the above-exemplified anti-FKN antibody (e.g., modification of the antibody, or partial substitution, addition or deletion of the amino acid sequence of the antibody), while retaining the function of the antibody, or in order to add or improve the function of the antibody, is also included in the antibody of the present invention. More specifically, an antibody whose lysine (Lys) positioned at the carboxy terminus (C-terminus) of a heavy chain has been deleted by an artificial method such as genetic modification to reduce the heterogeneity of an antibody generated from antibody-producing cells is also included in the scope of the present invention. Moreover, the anti-FKN antibody comprised in the pharmaceutical composition of the present invention does not necessarily have complete homogeneity, and thus, the present anti-FKN antibody includes, for example both an antibody that deletes lysine (Lys) positioned at the carboxy terminus (C-terminus) of a heavy chain thereof, and an antibody that does not delete such lysine, as long as it maintains the functions intended by the pharmaceutical composition of the present invention.

The anti-FKN antibody may be modified, as desired. The anti-FKN antibody may be modified to change (a) the three-dimensional structure of an amino acid sequence in a modified region, for example such as a sheet or helix conformation; (b) the charged or hydrophobic state of a molecule at a target site; or (c) the effect of modification on the maintenance of the volume of a side chain. Otherwise, it may also be a modification, in which such changes are not clearly observed.

Modification of the anti-FKN antibody may be achieved, for example, by substitution, deletion, addition or the like of constitutional amino acid residues.

In the present description, the term "amino acid" is used to have the most broad definition, and thus, examples of the amino acid include not only natural amino acids, such as serine (Ser), asparagine (Asn), valine (Val), leucine (Leu), isoleucine (Ile), alanine (Ala), tyrosine (Tyr), glycine (Gly), lysine (Lys), arginine (Arg), histidine (His), aspartic acid (Asp), glutamic acid (Glu), glutamine (Gln), threonine (Thr), cysteine (Cys), methionine (Met), phenylalanine (Phe), tryptophan (Trp), and proline (Pro), but also, unnatural amino acids, such as amino acid variants and derivatives. Taking into consideration such a broad definition of the amino acid, a person skilled in the art could naturally understand that examples of the amino acid mentioned in the present description include: L-amino acid; D-amino acid; chemically modified amino acids, such as amino acid variants and amino acid derivatives; amino acids that cannot be materials for constituting proteins *in vivo,* such as norleucine, β-alanine and ornithine; and chemically synthesized compounds having the properties of amino acids known to such a skilled person. Examples of the unnatural amino acid include **α**-methyl amino acids (α-methyl alanine, etc.), D-amino acids (D-aspartic acid, D-glutamic acid, etc.), histidine-like amino acids (2-amino-histidine, **β-**hydroxy-histidine, homohistidine, **α-**fluoromethyl-histidine, **α**-methyl-histidine, etc.), amino acids having redundant methylene on a side chain thereof ("homo" amino acid), and amino acids, the carboxylic acid functional group of which is substituted with a sulfonic acid group (cysteic acid, etc.).

Naturally existing amino acid residues can be classified into the following groups, for example, based on general side chain properties:
(1) hydrophobicity: Met, Ala, Val, Leu, Ile;
(2) neutral hydrophilicity: Cys, Ser, Thr;
(3) acidity: Asp, Glu;
(4) basicity: Asn, Gln, His, Lys, Arg;
(5) residues having influence on chain orientation: Gly, Pro; and
(6) aromaticity: Trp, Tyr, Phe.

Non-conservative substitution of amino acid sequences that constitute the anti-FKN antibody may be carried out by exchanging an amino acid belonging to one of these groups with another amino acid belonging to another group. More conservative substitution may be carried out by exchanging an amino acid belonging to one of these groups with another amino acid belonging to the same group. Likewise, deletion or substitution of an amino acid sequence may be carried out, as appropriate.

### 3. Pharmaceutical Composition and Formulation

The dosage form of the pharmaceutical composition of the present invention is not particularly limited, and it is typically a formulation prepared for use in intravenous administration (e.g., a formulation for injection).

In the case of the pharmaceutical composition of the present invention, the anti-FKN antibody, together with a pharmaceutically acceptable excipient, is added into, for example, water for injection, a normal saline, or a phosphate buffered saline, , such that it can be prepared in the form of a formulation for injection. Examples of the pharmaceutically acceptable excipient used in the present invention includea stabilizer, a surfactant, and a preservative. Representative excipients used for production of a formulation for injection and the production process thereof are known in the present technical field, and for example, Introduction to Pharmaceutical Dosage Forms, 1985, Ansel, H. C., Lea and Febiger, Philadelphia, Pa.; Remington's Pharmaceutical Sciences 1995, Mack Publ. Co., Easton, Pa. can be referred to. These publications are incorporated herein by reference in their entirety.

### 4. Dose, Dosing Intervals, and Number of Doses

The pharmaceutical composition or anti-FKN antibody of the present invention is administered to a human subject at a dose that provides to the subject, the serum concentration of an anti-FKN antibody that is therapeutically effective for Crohn's disease. For example, the pharmaceutical composition or anti-FKN antibody of the present invention is administered, such that the mean trough concentration of the anti-FKN antibody is 80 µg/mL or more.

In one embodiment of the present invention, the pharmaceutical composition or anti-FKN antibody of the present invention is used, such that the anti-FKN antibody is intravenously administered to a human at a dose of at least 10 mg/kg of human body weight. In another embodiment of the present invention, the pharmaceutical composition or anti-FKN antibody of the present invention is used, such that the anti-FKN antibody is intravenously administered to a human at a dose of 10 mg/kg of human body weight to 15 mg/kg of human body weight. In one embodiment of the present invention, the pharmaceutical composition or anti-FKN antibody of the present invention is used, such that the anti-FKN antibody is intravenously administered to a human at a dose of 10 mg/kg of human body weight or 15 mg/kg of human body weight.

The number of doses and the dosing intervals of the pharmaceutical composition or anti-FKN antibody of the present invention can be changed depending on the amount of the anti-FKN antibody in a single administration and the administration route.

In one embodiment of the present invention, the pharmaceutical composition or anti-FKN antibody of the present invention is used, such that the anti-FKN antibody is administered to a human subject in need thereof by multiple-dose intravenous administration, at a single dose of at least 10 mg/kg of human body weight, at dosing intervals from once every week to once two months. In another embodiment of the present invention, the pharmaceutical composition or anti-FKN antibody of the present invention is used, such that the anti-FKN antibody is administered to a human subject in need thereof by multiple-dose intravenous administration, at a single dose of at least 10 mg/kg of human body weight, at dosing intervals from once every week to once one month. In another embodiment of the present invention, the pharmaceutical composition or anti-FKN antibody of the present invention is used, such that the anti-FKN antibody is administered to a human subject in need thereof by multiple-dose intravenous administration, at a single dose of at least 10 mg/kg of human body weight, at dosing intervals from once every week to once every two weeks. In a specific embodiment of the present invention, the pharmaceutical composition or anti-FKN antibody of the present invention is used, such that the anti-FKN antibody is intravenously administered, at a single dose of at least 10 mg/kg of human body weight, at dosing intervals of once every week two times, and then at dosing intervals of every other week.

In another embodiment of the present invention, the pharmaceutical composition or anti-FKN antibody of the present invention is used, such that the anti-FKN antibody is administered to a human subject in need thereof by multiple-dose intravenous administration, at a single dose of 10 to 15 mg/kg of human body weight, at dosing intervals from once every week to once two months. In another embodiment of the present invention, the pharmaceutical composition or anti-FKN antibody of the present invention is used, such that the anti-FKN antibody is administered to a human subject in need thereof by multiple-dose intravenous administration, at a single dose of 10 to 15 mg/kg of human body weight, at dosing intervals from once every week to once one month. In another embodiment of the present invention, the pharmaceutical composition or anti-FKN antibody of the present invention is used, such that the anti-FKN antibody is administered to a human subject in need thereof by multiple-dose intravenous administration, at a single dose of 10 to 15 mg/kg of human body weight, at dosing intervals from once every week to once every two weeks. In a specific embodiment of the present invention, the pharmaceutical composition or anti-FKN antibody of the present invention is used, such that the anti-FKN antibody is intravenously administered to a human subject in need thereof, at a single dose of 10 to 15 mg/kg of human body weight, at dosing intervals of once every week two times, and then at dosing intervals of every other week.

In another embodiment of the present invention, the pharmaceutical composition or anti-FKN antibody of the present invention is used, such that the anti-FKN antibody is administered to a human subject in need thereof by multiple-dose intravenous administration, at a single dose of 10 mg/kg of human body weight, at dosing intervals from once every week to once two months. In another embodiment of the present invention, the pharmaceutical composition or anti-FKN antibody of the present invention is used, such that the anti-FKN antibody is administered to a human subject in need thereof by multiple-dose intravenous administration, at a single dose of 10 mg/kg of human body weight, at dosing intervals from once every week to once one month. In another embodiment of the present invention, the pharmaceutical composition or anti-FKN antibody of the present invention is used, such that the anti-FKN antibody is administered to a human subject in need thereof by multiple-dose intravenous administration, at a single dose of 10 mg/kg of human body weight, at dosing intervals from once every week to once every two weeks. In a specific embodiment of the present invention, the pharmaceutical composition or anti-FKN antibody of the present invention is used, such that the anti-FKN antibody is intravenously administered to a human subject in need thereof, at a dose of 10 mg/kg of human body weight, at dosing intervals of once every week two times, and then at dosing intervals of every other week.

In another embodiment of the present invention, the pharmaceutical composition or anti-FKN antibody of the present invention is used, such that the anti-FKN antibody is administered to a human subject in need thereof by multiple-dose intravenous administration, at a single dose of 15 mg/kg of human body weight, at dosing intervals from once every week to once two months. In another embodiment of the present invention, the pharmaceutical composition or anti-FKN antibody of the present invention is used, such that the anti-FKN antibody is administered to a human subject in need thereof by multiple-dose intravenous administration, at a single dose of 15 mg/kg of human body weight, at dosing intervals from once every week to once one month. In another embodiment of the present invention, the pharmaceutical composition or anti-FKN antibody of the present invention is used, such that the anti-FKN antibody is administered to a human subject in need thereof by multiple-dose intravenous administration, at a single dose of 15 mg/kg of human body weight, at dosing intervals from once every week to once every two weeks. In a specific embodiment of the present invention, the pharmaceutical composition or anti-FKN antibody of the present invention is used, such that the anti-FKN antibody is intravenously administered to a human subject in need thereof, at a dose of 15 mg/kg of human body weight, at dosing intervals of once every week two times, and then at dosing intervals of every other week.

The number of doses of the pharmaceutical composition or anti-FKN antibody of the present invention is not particularly limited, as long as it provides therapeutically effective improvement to Crohn's disease, and it can be changed depending on the amount of the anti-FKN antibody administered as a single dose, the administration route, and dosing intervals.

In an embodiment of the present invention, the pharmaceutical composition or anti-FKN antibody of the present invention can be intravenously administered to a subject at dosing intervals from once every week to once every two weeks, at least 2 times, at least 3 times, at least 4 times, at least 5 times, at least 6 times, at least 7 times, at least 8 times, at least 9 times, at least 10 times, at least 11 times, at least 12 times, at least 13 times, at least 14 times, at least 15 times, at least 16 times, at least 17 times, at least 18 times, at least 19 times, at least 20 times, at least 21 times, at least 22 times, at least 23 times, at least 24 times, at least 25 times, at least 26 times, at least 27 times, or more times, such that the mean trough concentration of the anti-FKN antibody is 80 µg/mL or more.

In one aspect, the present invention is a pharmaceutical composition for treating Crohn's disease, which comprises an anti-fractalkine antibody and a pharmaceutically acceptable excipient, wherein the pharmaceutical composition is characterized in that the anti-fractalkine antibody is an antibody comprising:
a heavy chain variable region comprising the amino acid sequence shown in SEQ ID NO: 13
a light chain variable region comprising the amino acid sequence shown in SEQ ID NO: 14 (DIQMTQSPSSLSASVGDRVTITCRASGNIHNFLAWYQQKPGKAPKLLIYNEKTLAD GVPSRFSGSGSGTDYTLTISSLQPEDFATYFCQQFWSTPYTFGGGTKVEIK); and
a constant region of human IgG2 isotype, wherein
the Fc region of the constant region of human IgG2 isotype comprises mutations V234A and G237A, and
the pharmaceutical composition is used, such that the anti-fractalkine antibody is intravenously administered to a human at a dose of at least 10 mg/kg of human body weight.

In another aspect, the present invention is a pharmaceutical composition for treating Crohn's disease, which comprises an anti-fractalkine antibody and a pharmaceutically acceptable excipient, wherein the pharmaceutical composition is characterized in that
the anti-fractalkine antibody is an antibody comprising:
a heavy chain variable region comprising the amino acid sequence shown in SEQ ID NO: 13
a light chain variable region comprising the amino acid sequence shown in SEQ ID NO: 14 (DIQMTQSPSSLSASVGDRVTITCRASGNIHNFLAWYQQKPGKAPKLLIYNEKTLAD GVPSRFSGSGSGTDYTLTISSLQPEDFATYFCQQFWSTPYTFGGGTKVEIK); and
a constant region of human IgG2 isotype, wherein
the Fc region of the constant region of human IgG2 isotype comprises mutations V234A and G237A, and
the pharmaceutical composition is used, such that the anti-fractalkine antibody is intravenously administered to a human at a dose of 10 to 15 mg/kg of human body weight.

In a further aspect, the present invention is a pharmaceutical composition for treating Crohn's disease, which comprises an anti-fractalkine antibody and a pharmaceutically acceptable excipient, wherein the pharmaceutical composition is characterized in that
the anti-fractalkine antibody is an antibody comprising:
a heavy chain variable region comprising the amino acid sequence shown in SEQ ID NO: 13
a light chain variable region comprising the amino acid sequence shown in SEQ ID NO: 14 (DIQMTQSPSSLSASVGDRVTITCRASGNIHNFLAWYQQKPGKAPKLLIYNEKTLAD GVPSRFSGSGSGTDYTLTISSLQPEDFATYFCQQFWSTPYTFGGGTKVEIK); and
a constant region of human IgG2 isotype, wherein
the Fc region of the constant region of human IgG2 isotype comprises mutations V234A and G237A, and
the pharmaceutical composition is used, such that the anti-fractalkine antibody is intravenously administered to a human at a dose of 10 mg/kg of human body weight.

In a further aspect, the present invention is a pharmaceutical composition for treating Crohn's disease, which comprises an anti-fractalkine antibody and a pharmaceutically acceptable excipient, wherein the pharmaceutical composition is characterized in that
the anti-fractalkine antibody is an antibody comprising:
a heavy chain variable region comprising the amino acid sequence shown in SEQ ID NO: 13
a light chain variable region comprising the amino acid sequence shown in SEQ ID NO: 14 (DIQMTQSPSSLSASVGDRVTITCRASGNIHNFLAWYQQKPGKAPKLLIYNEKTLAD GVPSRFSGSGSGTDYTLTISSLQPEDFATYFCQQFWSTPYTFGGGTKVEIK); and
a constant region of human IgG2 isotype, wherein
the Fc region of the constant region of human IgG2 isotype comprises mutations V234A and G237A, and
the pharmaceutical composition is used, such that the anti-fractalkine antibody is intravenously administered to a human at a dose of 15 mg/kg of human body weight.

In a preferred aspect, the present invention is a pharmaceutical composition for treating Crohn's disease, which comprises an anti-fractalkine antibody and a pharmaceutically acceptable excipient, wherein the pharmaceutical composition is characterized in that
the anti-fractalkine antibody is an antibody consisting of:
a heavy chain consisting of the amino acid sequence shown in SEQ ID NO: 11 (QVQLVQSGAEVKKPGASVKVSCKASGYTFTNYYIHWVKQAPGQGLEWIGWIYPGDG SPKFNERFKGRTTLTADKSTNTAYMLLSSLRSEDTAVYFCATGPTDGDYFDYWGQGT TVTVSSASTKGPSVFPLAPCSRSTSESTAALGCLVKDYFPEPVTVSWNSGALTSGVH TFPAVLQSSGLYSLSSVVTVPSSNFGTQTYTCNVDHKPSNTKVDKTVERKCCVECPP CPAPPAAAPSVFLFPPKPKDTLMISRTPEVTCVVVDVSHEDPEVQFNWYVDGVEVHN AKTKPREEQFNSTFRVVSVLTVVHQDWLNGKEYKCKVSNKGLPAPIEKTISKTKGQP REPQVYTLPPSREEMTKNQVSLTCLVKGFYPSDIAVEWESNGQPENNYKTTPPMLDS DGSFFLYSKLTVDKSRWQQGNVFSCSVMHEALHNHYTQKSLSLSPGK); and
a light chain consisting of the amino acid sequence shown in SEQ ID NO: 12 (DIQMTQSPSSLSASVGDRVTITCRASGNIHNFLAWYQQKPGKAPKLLIYNEKTLAD GVPSRFSGSGSGTDYTLTISSLQPEDFATYFCQQFWSTPYTFGGGTKVEIKRTVAAP SVFIFPPSDEQLKSGTASVVCLLNNFYPREAKVQWKVDNALQSGNSQESVTEQDSKD STYSLSSTLTLSKADYEKHKVYACEVTHQGLSSPVTKSFNRGEC), wherein
the pharmaceutical composition is used, such that the anti-fractalkine antibody is intravenously administered to a human at a dose of at least 10 mg/kg of human body weight.

In a preferred embodiment, the above described pharmaceutical composition is a pharmaceutical composition, which is administered to a human by multiple-dose intravenous administration at dosing intervals from once every week to once every two weeks.

According to the present invention, the above described pharmaceutical composition is a pharmaceutical composition, which is intravenously administered, such that the mean trough concentration of the above described anti-fractalkine antibody is 80 µg/mL or more.

In a preferred embodiment, the above described pharmaceutical composition is a pharmaceutical composition, wherein the above described anti-fractalkine antibody is intravenously administered to a human at a dose of 10 mg/kg of human body weight at dosing intervals of once every week three times, and then at dosing intervals of every other week at least 7 times.

In another aspect, the present invention is a pharmaceutical composition for use in a therapeutic method of Crohn's disease, wherein
the pharmaceutical composition comprises an anti-fractalkine antibody and a pharmaceutically acceptable excipient, wherein
the therapeutic method comprises administering the anti-fractalkine antibody to a human at a dose of at least 10 mg/kg of human body weight, wherein
the anti-fractalkine antibody is an antibody comprising:
a heavy chain variable region comprising the amino acid sequence shown in SEQ ID NO: 13
a light chain variable region comprising the amino acid sequence shown in SEQ ID NO: 14 (DIQMTQSPSSLSASVGDRVTITCRASGNIHNFLAWYQQKPGKAPKLLIYNEKTLAD GVPSRFSGSGSGTDYTLTISSLQPEDFATYFCQQFWSTPYTFGGGTKVEIK); and
a constant region of human IgG2 isotype, wherein
the Fc region of the constant region of human IgG2 isotype comprises mutations V234A and G237A.

In one embodiment of the present invention, the "human" is a Crohn's disease patient who has not obtained sufficient effects from corticosteroids or an immunomodulator, or who has not been continuous effects therefrom, or who has been intolerant thereto, or has not obtained sufficient effects from anti-TNF antibody.

The publications cited in the present description are provided only for the purpose of disclosing related art prior to the filing date of the present application.

The terms used in the present description are used to describe specific embodiments.

The terms "comprise" and "include" are used in the present description to intend that the described matter (a member, a step, an element, a number, etc.) is present, except in a case where it should be contextually apparently understood in a different way, and it does not exclude the presence of other matters (members, steps, elements, numbers, etc.). The term "consist of" includes the aspects indicated with the term "consist of" and/or "consist essentially of."

Unless otherwise specified, all of the terms used herein (including technical terms and scientific terms) have the same meanings as those that are broadly understood by a person skilled in the art to which the present invention pertains. Unless otherwise clearly specified, the terms used herein should be interpreted to have meanings that are consistent with the meanings in the present description and the related technical field, and the terms should not be interpreted to have ideal or excessively formal meanings.

The terms, such as a "first," a "second," and the like, are used to express various elements. It is understood that these elements should not be limited by these terms. These terms are only used to distinguish one element from another element, and thus, for example, it is possible to describe a first element as a second element, and likewise, to describe a second element as a first element, without deviating from the scope of the present invention.

In the present description, it should be understood that numerical values used to express the contents of components or numerical ranges and the like are modified with the term "approximately," unless otherwise clearly specified. For example, the term "10 µg" is understood to mean "approximately 10 µg," unless otherwise clearly specified. Accordingly, it is natural that a person killed in the art can theoretically understand the degree of a numerical value according to common technical knowledge and the context of the present description.

Except in a case where an aspect contextually apparently has another meaning, when an aspect that is given in a singular form is used in the present description and claims, it is understood that such an aspect may also have a plural form, and vice versa, unless it includes technical contradiction.

Hereinafter, the present invention will be described in more detail with reference to the following examples.

The abbreviations used in the following examples are commonly used abbreviations that are well known to a person skilled in the art. Several abbreviations will be described below.
AUC: area under the serum concentration-time curve
AUC_{(0-inf)} : area under the serum concentration-time curve from zero time extrap-olated to infinite time
AUC₍₀₋ₜ₎ : area under the concentration-time curve from zero time to time of last quantifiable concentration
AUC₍₀₋₃₃₆ₕ₎ : area under the concentration-time curve from zero (predose) to fixed time-point 336 h (2 weeks) after the end of infusion
CDAI: Crohn's disease activity index
CL: total clearance
CRP: C-reactive protein
Cₘₐₓ: maximum serum concentration
ELISA: enzyme-linked immunosorbent assay
FKN: fractalkine
IgG: immunoglobulin G
NONMEN: nonlinear mixed effect model
SAS: statistical analysis system
t_{1/2}: serum elimination half-life
tₘₐₓ: time to reach peak serum concentration
V_{d}: volume of distribution

### Examples

### Example 1: Preparation of humanized anti-human

### fractalkine antibody

In the following administration study in a human, a humanized anti-human fractalkine antibody H3-2L4 was used. Preparation of H3-2L4, which included humanization, was carried out in the same manner as that described in WO2011/052799. H3-2L4 used in Example 2 and the subsequent examples was prepared by the method described in the following 1-1. and 1-2.

### 1-1. Expression vector

An expression vector for the humanized anti-human fractalkine antibody (H3-2L4) was produced as follows.

First, a signal sequence (SEQ ID NO: 3) was added to the N-terminus of the amino acid sequence (SEQ ID NO: 1) of a heavy chain variable region (H3-2) of the humanized anti-human fractalkine antibody (H3-2L4), and the amino acid sequence (SEQ ID NO: 4) of the constant region of human IgG2, into which two mutations (V234A and G237A) had been inserted, was added to the C-terminus thereof (SEQ ID NO: 5). Subsequently, a signal sequence (SEQ ID NO: 6) was added to the N-terminus of the amino acid sequence (SEQ ID NO: 2) of a light chain variable region (L4) of the humanized anti-human fractalkine antibody (H3-2L4), and the amino acid sequence (SEQ ID NO: 7) of the constant region of human IgK was added to the C-terminus thereof (SEQ ID NO: 8). In order to allow these amino acid sequences (SEQ ID NOS: 5 and 8) to express in CHO cells, they were converted to optimal gene sequences. To the 5'-terminus of each gene sequence, a recognition sequence of the restriction enzyme HindIII and a Kozak sequence were added, and to the 3'-terminus thereof, a stop codon and a recognition sequence of the restriction enzyme EcoRI were added, so that the sequences were totally synthesized (SEQ ID NOS: 9 and 10).

It is to be noted that the sequences specified with SEQ ID NOS: 1 to 10 are as follows.

Amino acid sequence (SEQ ID NO: 1) of a heavy chain variable region (H3-2)

Amino acid sequence (SEQ ID NO: 2) of a light chain variable region (L4)

### Signal sequence (SEQ ID NO: 3)

### MEWSWVFLFFLSVTTGVHS

Amino acid sequence (SEQ ID NO: 4) of the constant region of human IgG2, into which two mutations (V234A and G237A) had been inserted

### Signal sequence (SEQ ID NO: 6)

### MSVPTQVLGLLLLWLTDARC

Amino acid sequence (SEQ ID NO: 7) of the constant region of human IgK

The totally synthesized gene sequence encoding a heavy chain was cleaved with the restriction enzymes HindIII and EcoRI, and it was then inserted into the HindIII-EcoRI site of a pEE6.4 vector (Lonza). The totally synthesized gene sequence encoding a light chain was cleaved with the restriction enzymes HindIII and EcoRI, and it was then inserted into the HindIII-EcoRI site of a pEE12.4 vector (Lonza). The two vectors were each cleaved with the restriction enzymes NotI and PvuI, and vector fragments each containing a heavy chain or a light chain were ligated to each other to construct an expression vector.

### 1-2. Construction of cell line expressing antibody H3-2L4, and obtainment of antibody H3-2L4

The constructed expression vector was introduced by electroporation into CHOK1SV cells (Lonza) conditioned in a medium of CD-CHO/6 mM L-glutamine. After completion of the gene introduction, selection was carried out in a medium of CD-CHO/50 uM MSX under an environment of 37°C/10% CO₂, to obtain cells expressing an antibody of interest. Thereafter, the cells were cloned to produce a cell bank. The produced cell bank was revived and cultured, and a culture supernatant was then purified by chromatography to obtain the antibody H3-2L4 of interest.

The antibody H3-2L4 obtained according to the above described operations had a heavy chain and a light chain having the amino acid sequences shown in SEQ ID NOS: 11 and 12, respectively.

Full-length heavy chain of H3-2L4 (SEQ ID NO: 11)

Full-length light chain of H3-2L4 (SEQ ID NO: 12)

Moreover, the amino acid sequences of the heavy chain variable region and the light chain variable region of the antibody H3-2L4 are as follows:

Heavy chain variable region of H3-2L4 (SEQ ID NO: 13)

Light chain variable region of H3-2L4 (SEQ ID NO: 14)

Furthermore, the amino acid sequences of the CDR-H1 to CDR-H3 and the CDR-L1 to CDR-L3 of the antibody H3-2L4 are as follows:
CDR-H1 of H3-2L4 (SEQ ID NO: 15)
   NYYIH
CDR-H2 of H3-2L4 (SEQ ID NO: 16)
   WIYPGDGSPKFNERFKG
CDR-H3 of H3-2L4 (SEQ ID NO: 17)
   GPTDGDYFDY
CDR-L1 of H3-2L4 (SEQ ID NO: 18)
   RASGNIHNFLA
CDR-L2 of H3-2L4(SEQ ID NO: 19)
   NEKTLAD
CDR-L3 of H3-2L4(SEQ ID NO: 20)
   QQFWSTPYT

### Example 2: Single-dose administration study

Healthy adult male subjects were used, and a phase I trial was carried out to evaluate safety, tolerability, and pharmacokinetics, when H3-2L4 was administered to the subjects by single-dose intravenous administration.

### 2-1. [Trial design]

The present trial was a single dose escalation study involving single institution, randomization, double blind and placebo control, which was carried out for the main purpose of evaluating safety and tolerability when H3-2L4 was administered to Japanese healthy adult male subjects by single-dose intravenous administration. In the present trial, 64 subjects were divided into 8 cohorts (0.0006, 0.006, 0.04, 0.2, 1, 3, 6, and 10 mg/kg groups), and thereafter, by single-dose intravenous administration, 6 subjects in each of the 8 cohorts were administered with H3-2L4, and 2 subjects therein were administered with a placebo.

The present trial was constituted with a screening period, an observation hospitalization period, an administration hospitalization period (double blind trial), a follow-up phase 1 period (double blind trial), and a follow-up phase 2 period (established for only Cohorts 5 to 8: non-blinded trial).

Within a period from 27 days to 2 days before administration of a trial drug, a screening test was carried out, and one day before the drug administration, a test performed in an observation hospitalization period was carried out, so as to confirm eligibility. Subjects whose eligibility had been confirmed were randomly assigned to an H3-2L4 group or a placebo group, based on an assignment list prepared by an assignment officer. With regard to two subjects in each cohort on day 1, one subject was assigned to H3-2L4 administration, and the other subject was assigned to placebo administration, followed by administration. The remaining six subjects were assigned to five H3-2L4 administration subjects and one placebo administration subject, and administration was carried out from day 2 to day 4. The number of subjects to which the drug was administered on the same day was set at 2, and two or more hours after initiation of the administration to one subject, the administration was carried out to the other subject. The trial drug was administered to the subjects by dissolving H3-2L4 or the placebo in a normal saline, and then administering the obtained solution to the subjects for approximately 30 minutes by intravenous drip infusion. The present trial was carried out by a single-dose administration, and administration to the subjects was carried out only once, regardless of their mealtime.

For one week after completion of the administration, the subjects were observed and inspected in the hospital, and thereafter, in the outpatient service, Cohorts 1 to 4 (0.0006, 0.006, and 0.04 mg/kg groups) were observed and inspected until 8 weeks after completion of the administration, and Cohorts 5 to 8 (0.2, 1, 3, 6, and 10 mg/kg groups) were observed and inspected until 24 weeks after completion of the administration.

Cohort migration was carried out based on the results of safety obtained from each cohort, when both a trial investigator and a trial client (a representative from a medical expert, a person in charge of safety department for trial clients, and a trial client) judged that there would be no problems regarding cohort migration. In principle, such cohort migration was carried out at an interval of 3 weeks after completion of the administration to the first subject in the previous cohort.

The types of the trial drugs used in the present study were as follows.

**[Table 1]**

| Table 1. Types of trial drugs | | |
|---|---|---|
| Type | Dosage form and content | Manufacturer |
| H3-2L4 | Aqueous solution containing 100 mg of H3-2L4 in 1 vial (10 mL) | Eisai Co., Ltd. |
| Placebo | Aqueous solution not containing H3-2L4 in 1 vial (10 mL) | |

### 2-2. [Administration of trial drugs]

H3-2L4 or placebo was diluted with a normal saline, depending on the body weight of each subject to adjust the amounts of liquids administered as shown in Table 2. Upon administration, employing a syringe pump or an infusion pump, each solution was administered to the subjects by intravenous drip infusion using an in-line filter (pore diameter: 0.2 µm) for approximately 30 minutes (including normal saline flushing).

**[Table 2]**

| **Table 2. Amounts of liquids administered and types of pumps** | | | | |
|---|---|---|---|---|
| Cohort | Trial drug | | Amount of liquid administered | Pump |
| 1 | H3-2L4 | 0.0006 mg/kg or placebo | 10 mL | Syringe pump |
| 2 | H3-2L4 | 0.006 mg/kg or placebo | 100 mL | Infusion pump |
| 3 | H3-2L4 | 0.04 mg/kg or placebo | | |
| 4 | H3-2L4 | 0.2 mg/kg or placebo | | |
| 5 | H3-2L4 | 1 mg/kg or placebo | | |
| 6 | H3-2L4 | 3 mg/kg or placebo | | |
| 7 | H3-2L4 | 6 mg/kg or placebo | | |
| 8 | H3-2L4 | 10 mg/kg or placebo | | |

### 2-3. [Analysis of serum concentration of H3-2L4]

The serum concentration of H3-2L4 was measured by a validated measurement method. Specifically, H3-2L4 in serum was allowed to bind to human fractalkine that was solid-phased on a microplate, and a ruthenium-labeled anti-H3-2L4 rabbit polyclonal antibody was then allowed to react therewith. Thereafter, the amount of electrochemical luminescence was measured using Sector Imager 6000 (Meso Scale Discovery) to quantify the serum concentration of H3-2L4. Blood to be used to measure the serum concentration of H3-2L4 was collected in blood collection timing shown in the following Table 3.

**[Table 3]**

| Table 3. Blood collection timing for evaluation of pharmacokinetics | | | | |
|---|---|---|---|---|
| Period | Blood collection timing (time elapsed after completion of administration on day 1) | | | Permissible range of blood collection time |
| Day 1 | Immediately before administration | | | -3 hours |
| | at the completion of administration (0 hours) | | | +5 minutes |
| | 1 hour | | | ±5 minutes |
| | 2 hours | | | |
| | 6 hours | | | |
| | 12 hours | | | |
| Day 2 | 24 hours | Same time as end time of administration on day 1 | | ±1 hour |
| Day 3 | 48 hours | | | |
| Day 5 | 96 hours | | | |
| Day 8 | 168 hours | | | |
| Day 15 | 336 hours | 2 weeks after | Same time as end time of administration on day 1 on same day of week as day 1 | ±24 hours |
| Day 22 | 504 hours | 3 weeks after | | |
| Day 29 | 672 hours | 4 weeks after | | |
| Day 36 | 840 hours | 5 weeks after | | |
| Day 43 | 1008 hours | 6 weeks after | | |
| Day 57 | 1344 hours | 8 weeks after | | |
| Day 71 | 1680 hours | 10 weeks after | | ±72 hours |
| Day 85 | 2016 hours | 12 weeks after | | |
| Day 99 | 2352 hours | 14 weeks after | | |
| Day 113 | 2688 hours | 16 weeks after | | |
| Day 127 | 3024 hours | 18 weeks after | | |
| Day 141 | 3360 hours | 20 weeks after | | |
| Day 155 | 3696 hours | 22 weeks after | | |
| Day 169 | 4032 hours | 24 weeks after | | |

### 2-4. [Analysis of pharmacokinetics]

Pharmacokinetics were analyzed based on the data regarding the serum concentration of H3-2L4, using a population of subjects having data from which one or more pharmacokinetic parameters can be calculated (hereinafter referred to as a "pharmacokinetics analysis target population"). In the present trial, with regard to 16 subjects to which placebo had been administered, 6 subjects to which 0.0006 mg/kg H3-2L4 had been administered, and 6 subjects to which 0.006 mg/kg H3-2L4 had been administered, the serum concentration of H3-2L4 of each subject was less than the lower quantification limit value (0.100 µg/mL). Accordingly, 36 subjects excluding the aforementioned subjects were determined to be a pharmacokinetics analysis target population. The summary statistics of the serum concentration of H3-2L4 was calculated for individual dosages in predetermined periods. A transition diagram of the serum concentration of H3-2L4 was prepared, and then, pharmacokinetic parameters including Cₘₐₓ, tₘₐₓ, AUC₍₀₋ₜ₎, AUC_{(0-inf)}, t_{1/2}, CL and V_{d} were calculated by a non-compartment analysis, using the serum concentration of H3-2L4. In addition, using the obtained Cₘₐₓ, AUC₍₀₋ₜ₎ and AUC_{(0-inf)}, dose proportionality was analyzed. The analysis was carried out using SAS, WinNonlin, Pharsight Knowledgebase Server, Microsoft Excel, and S-PLUS.

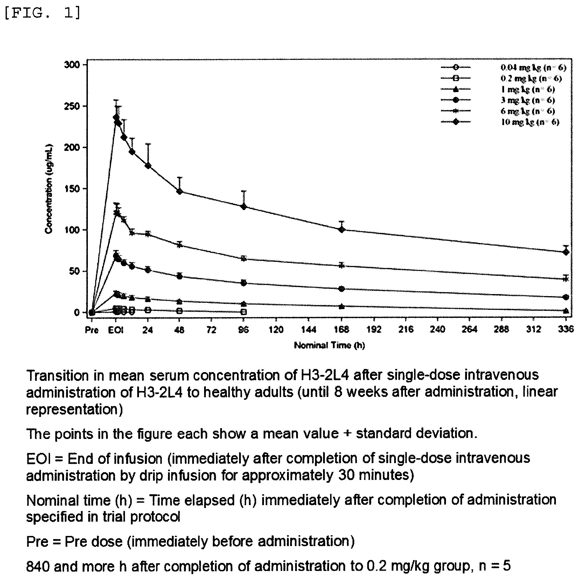

The thus calculated results are shown in the following Table 4 and FIG. 1. It is to be noted that the mean values of the pharmacokinetic parameters shown in Table 4, except for the tₘₐₓ, are shown in the form of arithmetical mean values.

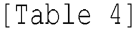

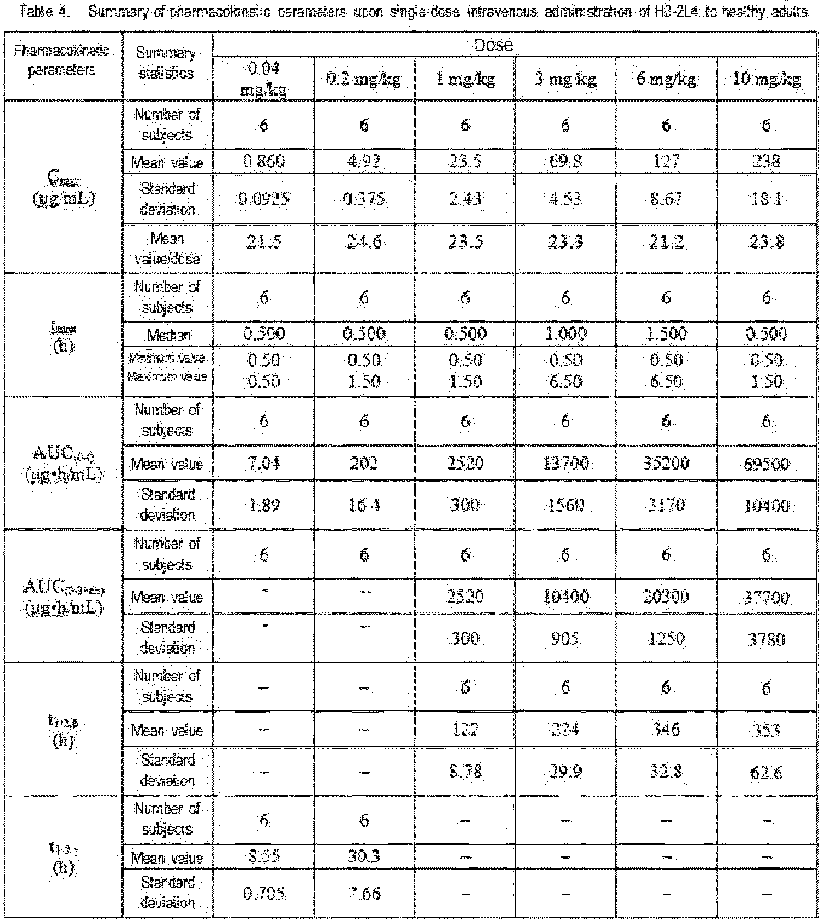

As a result of the analysis, it was considered that the pharmacokinetics obtained by single-dose intravenous administration of H3-2L4 would show a three-phase profile consisting of phase α, phase β and phase γ in 0.04 to 10 mg/kg. After completion of the intravenous drip infusion, an initial distribution phase that was promptly reduced from the Cₘₐₓ (phase **α**) was present, and thereafter, a slowly disappearing phase (phase **β**) was observed in a high concentration, and a rapid final disappearing phase (phase γ) was observed in a low concentration. The boundary point between the phase γ and the phase **β** was assumed to be around 10 µg/mL. In the 0.04 and 0.2 mg/kg groups, a two-phase profile was found, and it was considered that the phase γ would appear following the phase α. In the 1 to 10 mg/kg groups, since the number of collected blood samples necessary for precise evaluation of the phase γ as a finally disappearing phase was insufficient, phase γ-dependent pharmacokinetic parameters could not be calculated. After completion of the intravenous drip infusion for 30 minutes, almost all of the subjects reached the tₘₐₓ after 0.5 hours, but some subjects reached the tₘₐₓ after 6.5 hours. The t_{1/2}, **β** was longer than the t_{1/2},γ, and it was prolonged with an increase in the dose, but the t_{1/2},**β** in the 6 and 10 mg/kg groups became almost the same, and thus, it was considered that it would reach a plateau. The Cₘₐₓ was increased in proportion to the dose in 0.04 to 10 mg/kg groups. However, the AUC₍₀₋ₜ₎ and AUC_{(0-inf)} were not in proportion to the dose, and H3-2L4 showed non-linear pharmacokinetics.

### Example 3: Multiple-dose administration study

A phase I/II clinical study was carried out by repeated intravenous administration of H3-2L4 to Crohn's disease patients used as subjects, so as to evaluate safety, tolerability, pharmacokinetics, and efficacy on Crohn's disease.

### 3-1. [Trial design]

The present trial was a multiple ascending dose (MAD) study involving multi-institutional joint, non-blind and non-control, which was carried out for the main purpose of evaluating safety and tolerability when H3-2L4 was administered to Japanese Crohn's disease patients used as subjects by repeated intravenous administration. In the present trial, randomization was not carried out. In the present trial, 21 subjects were divided by 6 subjects, 8 subjects, and 7 subjects into Cohorts 1 to 3 (2, 5, and 10 mg/kg groups), respectively. Thereafter, H3-2L4 was administered to the subjects by repeated intravenous administration. Moreover, regarding Cohort 4 (15 mg/kg group), H3-2L4 was administered to at least 6 subjects by repeated intravenous administration.

The present trial was constituted with a screening period, an observation period, an administration period, a continuous administration period, and a follow-up period.

A screening test was carried out within 42 to 2 days before initiation of the administration of the trial drug, and an observation period test was carried out one day before initial administration of the trial drug or before the administration on the administration day. To subjects who were confirmed to have eligibility, H3-2L4 was administered.

Selection criteria for eligibility were as follows:
(1) Patients who are 20 years old or older and younger than 65 years old;
(2) Patients who are diagnosed to have Crohn's disease in accordance with the Crohn's disease diagnosis criteria (2012) by the "Research Study regarding Refractory Inflammatory Bowel Disease" Team, Health Labour Sciences Research Grant Study for Refractory Diseases;
(3) Patients whose severity in the observation period is a mild to medium degree (CDAI is 150 or more and less than 450, based on the Crohn's disease diagnosis criteria); and
(4) Patients who were administered with a 5-aminosalicylic acid (5-ASA) formulation, salazosulfapyridine, adrenocorticosteroid, an immunomodulator, infliximab (including biosimilars, and so on) or adalimumab in the past, and were not found to have effects, or patients who were found to have effects, but after that, the effects were attenuated or disappeared, or patients to which administration could not be continued due to side effects (except for infliximab and adalimumab).

H3-2L4 was dissolved in approximately 100 mL of normal saline, and was then administered to the patients for approximately 30 minutes by intravenous drip infusion. In the administration period, with regard to Cohort 1 (2 mg/kg) and Cohort 2 (5 mg/kg), H3-2L4 was administered to the patients every two weeks until 10 weeks in a total of 6 times (two times amount in 0 week), and with regard to Cohort 3 (10 mg/kg) and Cohort 4 (15 mg/kg), H3-2L4 was administered to the patients at the 0th week, 1st week, 2nd week, and then every two weeks until the 10th week in total of 7 times. In the evaluation carried out at the 12th week, the subjects who had no problems regarding safety, had a Crohn's disease activity index (CDAI) of less than 150 or a reduction in the CDAI from the observation period that is 70 or more, and wanted continuous administration, were further administered with H3-2L4 at the same dose as described above, every two weeks, 20 times (40 weeks) (continuous administration period). Regarding the continuous administration period, the administration could be changed (reduced) to administration at the same dose as described above every four weeks according to the judgement of a trial investigator or a doctor in charge of the trial (wherein it is also possible to return the administration to an administration every two weeks).

Cohort migration was carried out based on the results of safety obtained from each cohort, when both a trial investigator and a trial client judged that there would be no problems regarding cohort migration.

The type of the trial drug used in the present study was as follows.

**[Table 5]**

| Table 5. Trial drug | | |
|---|---|---|
| Type | Dosage form and content | Manufacturer |
| H3-2L4 | Aqueous solution containing 100 mg of H3-2L4 in 1 vial (1 mL) | Eisai Co., Ltd. |

### 3-2. [Administration of trial drug]

H3-2L4 was diluted with a normal saline, depending on the body weight of each subject, so as to adjust the administered amount of a liquid containing each dose of H3-2L4 (approximately 100 mL). Individual doses (2, 5, 10 and 15 mg/kg) and the administration method are as shown in Table 6. The body weight used to calculate the dose (total amount) relative to body weight was measured in the observation period, the 12th week, the 24th week, and the 36th week, and even if the body weight was increased or decreased during other periods, the relative dose (total amount) was not changed. Upon administration, employing an infusion pump, each solution was administered to the subjects by intravenous drip infusion using an in-line filter (pore diameter: 0.2 µm) for approximately 30 minutes (including normal saline flushing). It is to be noted that the trial drug could be administered regardless of their mealtime.

**[Table 6]**

| Table 6. Dose of H3-2L4 and administration method | | | | |
|---|---|---|---|---|
| Cohort | Trial drug | | Amount of liquid administered | Pump |
| 1 | H3-2L4 | 2 mg/kg | Approx. 100 mL | Infusion pump |
| 2 | H3-2L4 | 5 mg/kg | | |
| 3 | H3-2L4 | 10 mg/kg | | |
| 4 | H3-2L4 | 15 mg/kg | | |

### 3-3. [Pharmacokinetics and pharmacodynamics]

The serum concentration of H3-2L4 and the serum concentration of total FKN were measured by validated measurement methods. The serum concentration of H3-2L4 was measured by the method described in 2-4 of Example 2. The serum concentration of total FKN was measured by the sandwich ELISA method described in WO2011/052799, involving sandwiching with two types of anti-human fractalkine monoclonal antibodies 1F3 and 3A5-2. Specifically, total FKN in the serum was captured by 1F3 that was solid-phased on a plate, it was then sandwiched with 3A5-2 labeled with horse radish peroxidase (HRP), it was further reacted with 3,3',5,5'-tetramethylbenzidine (TMB) as a substrate of HRP, and the coloration value was then measured using an auto-reader (Microplate reader Envision, Perkin Elmer Japan), so that the serum concentration of total FKN was quantified.

Pharmacokinetics were analyzed based on the data of the serum concentration of H3-2L4, using a population of subjects who were administered with the trial drug, and as a result, had one or more of serum H3-2L4 concentration data to be evaluated (hereinafter referred to as a "pharmacokinetics analysis target population"). The summary statistics of the serum concentration of H3-2L4 was calculated for individual dosages in predetermined periods, and a transition diagram of the serum concentration of H3-2L4 was prepared.

Pharmacodynamics were analyzed using a population of subjects who were administered with the trial drug, and as a result, had one or more of pharmacodynamic biomarker data to be evaluated (hereinafter referred to as a "pharmacodynamics analysis target population"). With regard to the measurement values of the serum concentration of total FKN, the summary statistics was calculated for individual dosages in predetermined evaluation periods.

### 3-3-1. [Serum concentration of H3-2L4 and serum concentration of total FKN]

From the time of administration of the trial drug until the 12th week, blood was collected to measure the serum concentration of H3-2L4 and the serum concentration of total FKN, within 10 minutes before and after administration (0 hour), in the periods indicated with the letters X, shown in the timeline of Table 7 for Cohorts 1 and 2, and of Table 8 for Cohorts 3 and 4. With regard to the subjects who were migrated in the continuous administration period, blood was further collected every four weeks until the 52nd week. Moreover, the subjects, who were withdrawn from the trial during the administration period or the continuous administration period, were subjected to blood collection at the time of withdrawal.

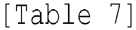

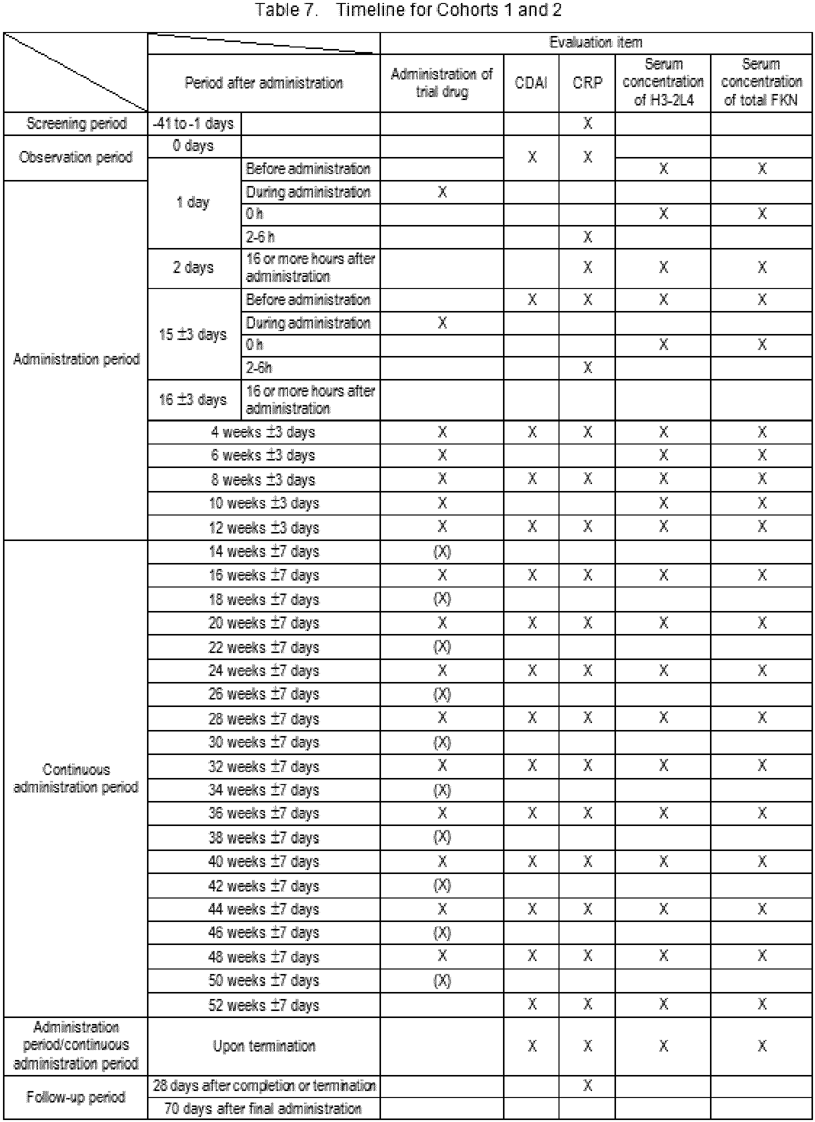

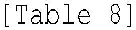

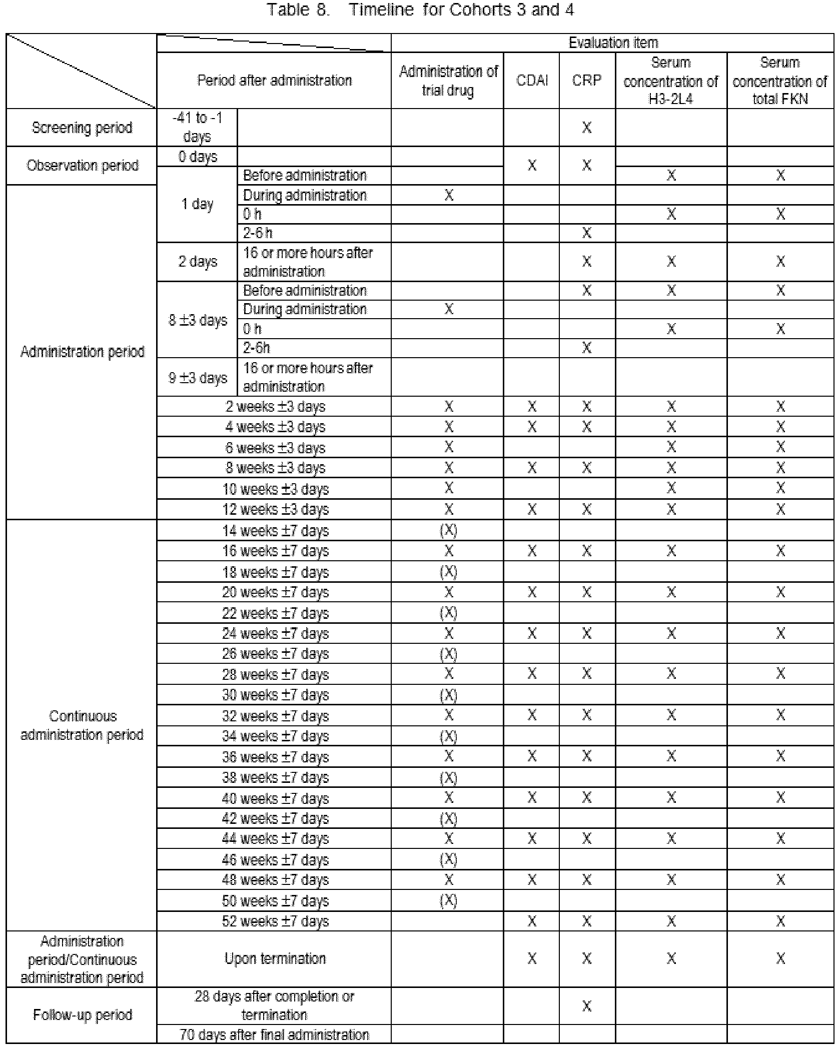

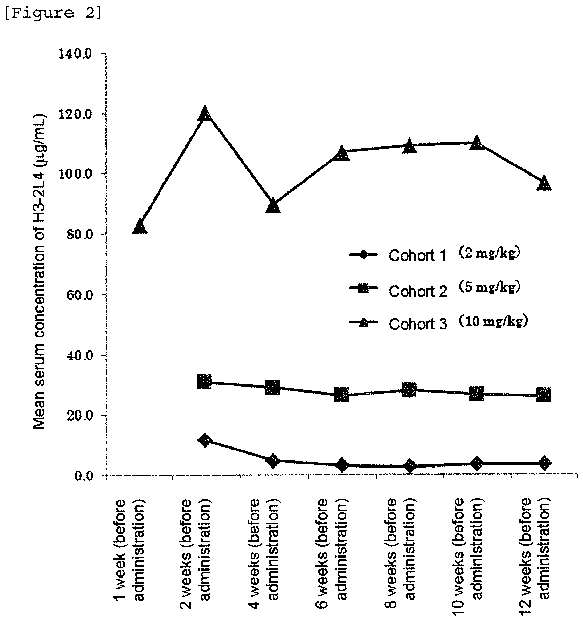

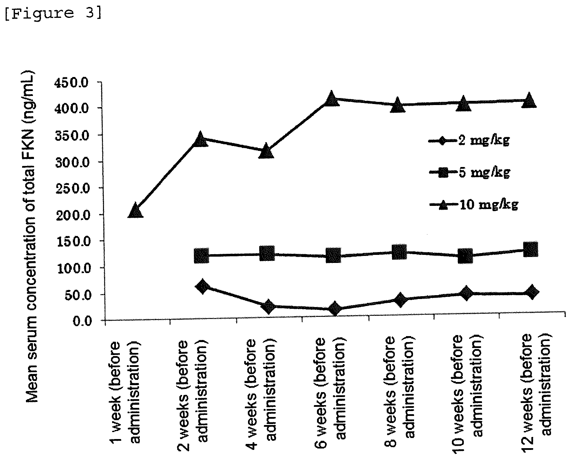

The mean values of the serum concentration of H3-2L4 and the serum concentration of total FKN in a 2 mg/kg administration group, a 5 mg/kg administration group, and a 10 mg/kg administration group over the administration period (12 weeks) (wherein the mean value was indicated as an arithmetical mean value) are shown in Table 9 and Table 10, respectively. In addition, transitions in the mean values of the serum concentration of H3-2L4 and the serum concentration of total FKN are shown in FIG. 2 and FIG. 3, respectively.

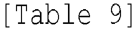

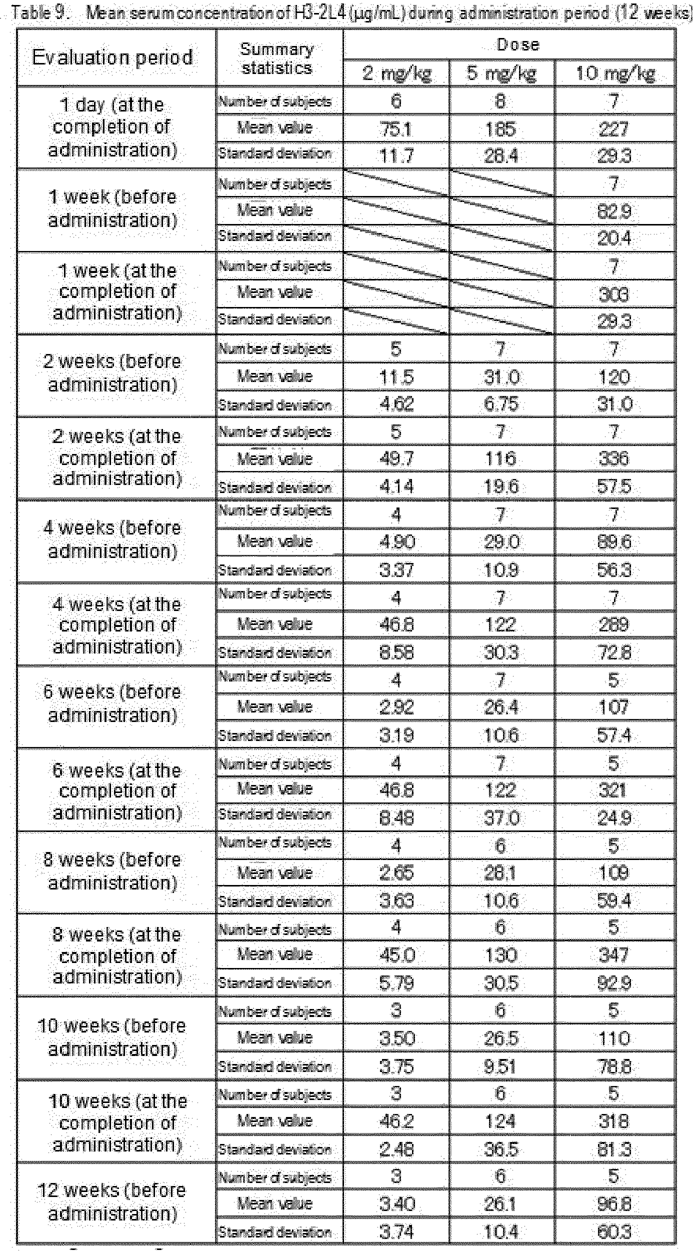

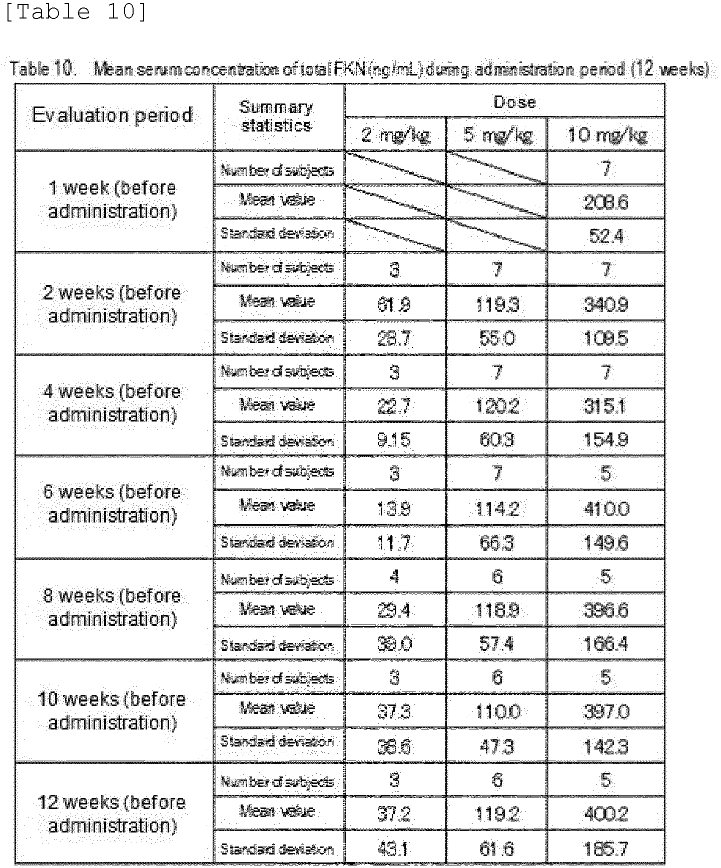

### 3-4. [Efficacy]

Efficacy was analyzed using a population of subjects who were administered with the trial drug, and as a result, had one or more of efficacy data after completion of the administration of the trial drug to be evaluated (FAS: Full Analysis Set).

The summary statistics of CDAI and CRP were calculated for individual dosages in predetermined evaluation periods.

The analysis was carried out using WinNonlin, NONMEM, SAS, etc.

### [CDAI]

CDAI was calculated from the subject diary, the corresponding test results, etc., in the periods indicated with the letters X shown in the timelines (Table 7 and Table 8). Moreover, with regard to the subjects who were migrated in the continuous administration period, CDAI was further calculated every four weeks until the 52nd week in the same manner as that described above. Upon the calculation of CDAI, regarding hematocrit and body weight, the values obtained on the day of evaluation were used, whereas regarding body height, the most recent value, which was measured according to the timeline, was used.

When the CDAI score was reduced by 70 or more, from that in the observation period, it was defined as "improvement," and when the CDAI score was less than 150, it was defined as "remission."

The number of subjects in Cohorts 1 to 3 at the time of initiation of the present trial was 6 subjects, 8 subjects and 7 subjects, respectively. Until the 12th week after the first administration, the trial performed on 3 subjects, 2 subjects and 2 subjects, respectively, in Cohorts 1 to 3, was terminated. It is to be noted that the subjects in Cohorts 1 to 3 had previously used the anti-TNF antibody, except for one subject in Cohort 2. The subjects who had used one agent were 2 subjects, 5 subjects and 3 subjects, respectively, in Cohorts 1 to 3, whereas the subjects who had used two agents were 4 subjects, 2 subjects and 4 subjects, respectively. In all of these subjects, the effects obtained by the use of the anti-TNF antibody were insufficient.

Among the subjects having a CDAI score of 220 or more before administration of the trial drug, the subjects who were confirmed to reduce the CDAI score by 70 or more (CR-70) in the evaluation at the time of the 12th week, using the CDAI score before administration of the trial drug as a baseline, were 1 subject, 2 subjects and 4 subjects, respectively, in Cohorts 1 to 3. Moreover, a reduction in the CDAI score of 100 or more (CR-100) was observed in 1 subject, 1 subject and 4 subjects, respectively, in Cohorts 1 to 3. Furthermore, 3 subjects in Cohort 3 achieved remission.

Further, the subjects who had completed the continuous administration period were 1 subject, 2 subjects and 2 subjects, respectively, in Cohorts 1 to 3. In the evaluation at the 52nd week, 1 subject, 1 subject and 2 subjects respectively in Cohorts 1 to 3 were found to have a reduction in the CDAI score of 100 or more (CR-100), using the CDAI score before administration of the trial drug as a baseline. Still further, 2 subjects in Cohort 3 achieve remission.

Further, in the evaluation at the 12th week, the improvement effects were observed also in Cohort 4, and subjects who were migrated to the continuous administration period were found.

### [CRP]

CRP was measured in the periods indicated with the letters X shown in the timelines (Table 7 and Table 8).

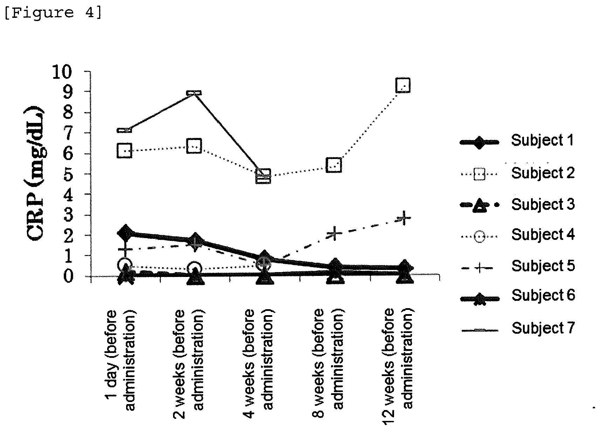

Transitions in the CRP values of 7 subjects in Cohort 3 over 12 weeks are shown in FIG. 4. Three subjects (subjects 1, 3 and 6), who had achieved remission based on the CDAI scores in the evaluation at the 12th week, were also found to have low CRP values.

## Claims

1. A pharmaceutical composition for use in treating Crohn's disease, which comprises an anti-fractalkine antibody and a pharmaceutically acceptable excipient, wherein
the anti-fractalkine antibody is an antibody comprising:
a heavy chain variable region comprising the amino acid sequence shown in SEQ ID NO: 13
a light chain variable region comprising the amino acid sequence shown in SEQ ID NO: 14
a constant region of human IgG2 isotype, wherein
the Fc region of the constant region of human IgG2 isotype comprises mutations V234A and G237A, and
the anti-fractalkine antibody is intravenously administered to a human at a dose of at least 10 mg/kg of human body weight and the mean trough concentration of the anti-fractalkine antibody is 80 µg/mL or more.

2. The pharmaceutical composition for use according to claim 1,
wherein
the anti-fractalkine antibody is intravenously administered to a human at a dose of 10 to 15 mg/kg of human body weight.

3. The pharmaceutical composition for use according to claim 1, wherein
the anti-fractalkine antibody is intravenously administered to a human at a dose of 10 mg/kg of human body weight.

4. The pharmaceutical composition for use according to claim 1, wherein
the anti-fractalkine antibody is intravenously administered to a human at a dose of 15 mg/kg of human body weight.

5. The pharmaceutical composition for use according to any one of claims 1 to 4, which is administered by multiple-dose intravenous administration at dosing intervals from once every week to once every two weeks.

## Patentansprüche

1. Pharmazeutische Zusammensetzung zur Verwendung bei der Behandlung von Morbus Crohn, welche einen Anti-Fraktalkin-Antikörper und einen pharmazeutisch akzeptablen Hilfsstoff umfasst, wobei der Anti-Fraktalkin-Antikörper ein Antikörper ist, umfassend:
eine variable Region der schweren Kette, umfassend die in SEQ ID NO: 13 gezeigte Aminosäuresequenz
eine variable Region der leichten Kette, umfassend die in SEQ ID NO: 14 gezeigte Aminosäuresequenz und
eine konstante Region des menschlichen IgG2-Isotyps, wobei die Fc-Region der konstanten Region des humanen IgG2-Isotyps die Mutationen V234A und G237A umfasst, und der Anti-Fraktalkin-Antikörper einem Menschen in einer Dosis von mindestens 10 mg/kg des menschlichen Körpergewichts intravenös verabreicht wird und die mittlere Minimalkonzentration (through concentration) des Anti-Fraktalkin-Antikörpers 80 µg/mL oder mehr beträgt.

2. Pharmazeutische Zusammensetzung zur Verwendung gemäß Anspruch 1, wobei der Anti-Fraktalkin-Antikörper einem Menschen intravenös in einer Dosis von 10 bis 15 mg/kg des menschlichen Körpergewichts verabreicht wird.

3. Pharmazeutische Zusammensetzung zur Verwendung gemäß Anspruch 1, wobei der Anti-Fraktalkin-Antikörper einem Menschen intravenös in einer Dosis von 10 mg/kg des menschlichen Körpergewichts verabreicht wird.

4. Pharmazeutische Zusammensetzung zur Verwendung gemäß Anspruch 1, wobei der Anti-Fraktalkin-Antikörper einem Menschen intravenös in einer Dosis von 15 mg/kg des menschlichen Körpergewichts verabreicht wird.

5. Pharmazeutische Zusammensetzung zur Verwendung gemäß irgendeinem der Ansprüche 1 bis 4, welche durch intravenöse Verabreichung von Mehrfachdosen in Dosierungsintervallen von einmal pro Woche bis einmal alle zwei Wochen verabreicht wird.

## Revendications

1. Composition pharmaceutique destinée à être utilisée dans le traitement de la maladie de Crohn, qui comprend un anticorps anti-fractalkine et un excipient pharmaceutiquement acceptable, dans laquelle
l'anticorps anti-fractalkine est un anticorps comprenant :
une région variable de chaîne lourde comprenant la séquence d'acides aminés représentée dans SEQ ID No : 13
une région variable de chaîne légère comprenant la séquence d'acides aminés représentée dans SEQ ID No : 14
une région constante d'isotype IgG2 humain, dans laquelle
la région Fc de la région constante d'isotype IgG2 humain comprend des mutations V234A et G237A et
l'anticorps anti-fractalkine est administré par voie intraveineuse à un être humain à une dose d'au moins 10 mg/kg de poids corporel humain et une concentration minimale moyenne de l'anticorps anti-fractalkine est de 80 µg/ml ou plus.

2. Composition pharmaceutique destinée à être utilisée selon la revendication 1, dans laquelle
l'anticorps anti-fractalkine est administré par voie intraveineuse à un être humain à une dose de 10 à 15 mg/kg de poids corporel humain.

3. Composition pharmaceutique destinée à être utilisée selon la revendication 1, dans laquelle
l'anticorps anti-fractalkine est administré par voie intraveineuse à un être humain à une dose de 10 mg/kg de poids corporel humain.

4. Composition pharmaceutique destinée à être utilisée selon la revendication 1, dans laquelle
l'anticorps anti-fractalkine est administré par voie intraveineuse à un être humain à une dose de 15 mg/kg de poids corporel humain.

5. Composition pharmaceutique destinée à être utilisée selon l'une quelconque des revendications 1 à 4, qui est administrée par une administration intraveineuse à doses multiples à des intervalles de une fois chaque semaine à une fois toutes les deux semaines.
